(19) Europäisches Patentamt / European Patent Office / Office européen des brevets

(11) **EP 3 245 946 B1**

(12) **FASCICULE DE BREVET EUROPEEN**

(45) Date de publication et mention de la délivrance du brevet:
**20.02.2019 Bulletin 2019/08**

(51) Int Cl.:
*A61B 5/00* (2006.01)  *A61B 5/04* (2006.01)
*G06N 3/04* (2006.01)

(21) Numéro de dépôt: **17171730.9**

(22) Date de dépôt: **18.05.2017**

(54) **PROCÉDÉ DE TRI NON SUPERVISÉ EN TEMPS RÉEL DE POTENTIELS D'ACTION D'UNE PLURALITÉ DE NEURONES BIOLOGIQUES**

NICHT ÜBERWACHTES ECHTZEIT-SORTIERVERFAHREN VON AKTIONSPOTENZIALEN EINER VIELZAHL VON BIOLOGISCHEN NEURONEN

METHOD FOR UNSUPERVISED REAL-TIME SORTING OF ACTION POTENTIALS OF A PLURALITY OF BIOLOGICAL NEURONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.05.2016 FR 1654485**

(43) Date de publication de la demande:
**22.11.2017 Bulletin 2017/47**

(73) Titulaires:
- **Commissariat à l'Energie Atomique et aux Energies Alternatives**
  **75015 Paris (FR)**
- **Institut National de la Santé et de la Recherche Médicale**
  **75013 Paris (FR)**

(72) Inventeurs:
- **BERNERT, Marie**
  **38000 Grenoble (FR)**
- **VIANELLO, Elisa**
  **38000 Grenoble (FR)**
- **YVERT, Blaise**
  **38120 Saint Egrève (FR)**

(74) Mandataire: **Cabinet Camus Lebkiri**
  **25 rue de Maubeuge**
  **75009 Paris (FR)**

(56) Documents cités:
**WO-A1-2015/184328    WO-A2-2014/025765
US-A1- 2009 216 091**

- BEINUO ZHANG ET AL: "A neuromorphic neural spike clustering processor for deep-brain sensing and stimulation systems", 2015 IEEE/ACM INTERNATIONAL SYMPOSIUM ON LOW POWER ELECTRONICS AND DESIGN (ISLPED), 1 juillet 2015 (2015-07-01), pages 91-97, XP055342652, DOI: 10.1109/ISLPED.2015.7273496 ISBN: 978-1-4673-8009-6
- FEDERICO CORRADI ET AL: "A Neuromorphic Event-Based Neural Recording System for Smart Brain-Machine-Interfaces", IEEE TRANSACTIONS ON BIOMEDICAL CIRCUITS AND SYSTEMS, vol. 9, no. 5, 26 octobre 2015 (2015-10-26), pages 699-709, XP055236096, US ISSN: 1932-4545, DOI: 10.1109/TBCAS.2015.2479256

**Description**

## DOMAINE TECHNIQUE DE L'INVENTION

Le domaine technique de l'invention est celui du tri de potentiels d'action de neurones biologiques. La présente invention concerne un procédé de tri non supervisé en temps réel de potentiels d'actions d'une pluralité de neurones biologiques.

Un domaine d'application de la présente invention est notamment celui des neuroprothèses et interfaces cerveau-machine (ou « brain-computer interfaces » en anglais), c'est-à-dire des interfaces de communication directe entre un cerveau et un dispositif externe tel qu'un ordinateur. Une interface cerveau-machine exploite les propriétés électriques de neurones et peut être conçue pour assister, améliorer ou réparer des fonctions humaines d'action défaillantes.

## ARRIERE-PLAN TECHNOLOGIQUE DE L'INVENTION

En neurobiologie, une microélectrode est un élément d'un dispositif permettant de mesurer une différence de potentiel électrique entre deux points d'un système biologique, par exemple :

- entre l'intérieur d'un neurone et un point de référence : il s'agit alors d'une microélectrode intracellulaire, ou
- entre un point proche d'un neurone et un point de référence : il s'agit alors d'une microélectrode extracellulaire.

Le type de signal obtenu en mesurant le champ électrique généré par un neurone depuis l'intérieur dudit neurone est très différent de celui obtenu en mesurant ledit champ électrique depuis l'extérieur dudit neurone.

Lorsqu'on enregistre une activité neuronale au moyen d'une microélectrode extracellulaire, la différence de potentiel mesurée reflète les courants traversant la membrane des neurones situés à proximité de ladite microélectrode extracellulaire. Ces courants membranaires comprennent :

- des courants membranaires avec des variations rapides, c'est-à-dire à des fréquences de l'ordre de 1 kHz, qui sous-tendent la création et la propagation de potentiels d'action (« action potentials » or « spikes » en anglais), et
- des courants membranaires avec des variations plus lentes, c'est-à-dire à des fréquences dans l'intervalle allant de 0 Hz à 500 Hz, appelés potentiels de champ locaux LFP (de l'anglais « local field potentials »), qui sous-tendent principalement les activités synaptiques.

En réalisant un filtrage passe-bande du signal brut enregistrant la totalité de l'activité neuronale, on peut isoler l'activité neuronale correspondant uniquement aux potentiels d'action. Le filtrage passe-bande est par exemple réalisé entre 300 Hz et 3 kHz lorsqu'on utilise l'activité neuronale liée aux potentiels d'action pour étudier la dynamique d'ensembles de neurones, et pour des applications d'interfaces cerveau-machine.

Quand un neurone suffisamment proche de la pointe d'une microélectrode extracellulaire émet un potentiel d'action, la microélectrode extracellulaire enregistre ce potentiel d'action. La pointe d'une microélectrode extracellulaire étant généralement à proximité de plusieurs neurones, le signal enregistré par la microélectrode extracellulaire peut comprendre une superposition de potentiels d'action générés par plusieurs neurones actifs différents. Un neurone donné émet typiquement un potentiel d'action pour un certain type de comportement ou d'expérience sensorielle de la part d'un individu. Par exemple, un premier neurone du cortex moteur peut émettre un potentiel d'action pour un mouvement du bras vers la gauche alors qu'un deuxième neurone du cortex moteur, voisin du premier neurone, émet quant à lui un potentiel d'action pour un mouvement du bras vers la droite. Il est ainsi très important de séparer, ou trier, les potentiels d'action propres à chaque neurone à partir du signal brut enregistré par une microélectrode extracellulaire. Un tel tri de potentiels d'actions est typiquement réalisé en utilisant le fait que chaque neurone émet sur une microélectrode donnée des potentiels d'action ayant une forme spécifique. La forme spécifique, sur une microélectrode donnée, des potentiels d'action émis par chaque neurone dépend notamment de la géométrie du neurone et de la position du neurone par rapport à la microélectrode.

Dans le contexte des interfaces cerveau-machine où l'on cherche à décoder un signal neuronal pour prédire un comportement, comme par exemple un mouvement, on obtient de meilleurs résultats avec des données de potentiels d'action triées qu'avec des données de potentiels d'action non triées. On obtient également des résultats d'autant plus précis qu'on enregistre l'activité d'un grand nombre de neurones. Les techniques d'enregistrement actuelles permettent d'utiliser simultanément des centaines de microélectrodes.

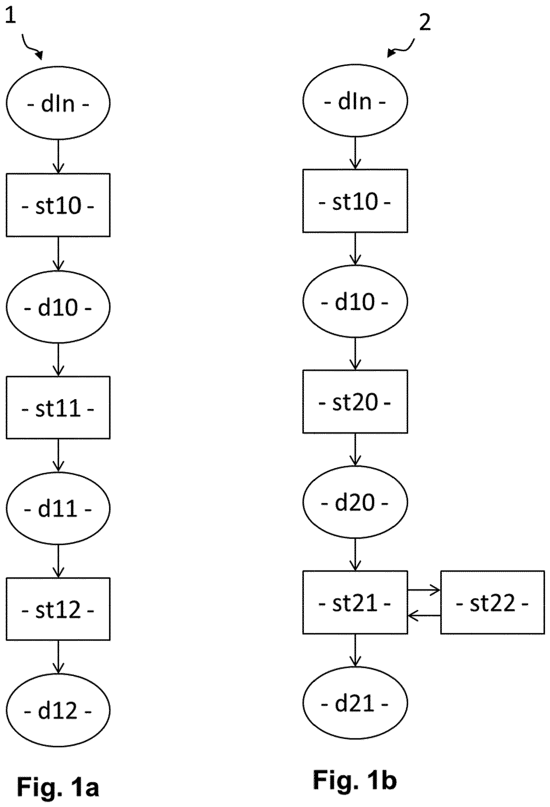

Plusieurs méthodes de tri de potentiels d'action en différé (« offline » en anglais), sont connues, selon lesquelles le signal brut issu de la ou des microélectrodes est d'abord entièrement enregistré et stocké avant d'être traité. Une méthode de tri en différé peut mettre en oeuvre des calculs longs ou recourir à la supervision d'un opérateur humain. Ce n'est pas le cas d'une méthode de tri en temps réel, qui doit pouvoir traiter le signal brut à chaque instant, au fur et à mesure. La figure 1a montre un diagramme des étapes d'un premier procédé 1 de tri de potentiels d'action selon l'art antérieur. Le premier procédé 1 comporte :

- à partir de données d'entrée dln, une première étape st10 de détection des potentiels d'action et de stockage des formes d'onde des potentiels d'action détectés d10 ;
- à partir des formes d'onde des potentiels d'action détectés d10, une deuxième étape st11 d'extraction de caractéristiques pertinentes d11 ;
- à partir des caractéristiques pertinentes d11, une troisième étape st12 de regroupement (« clustering » en anglais) desdites caractéristiques pertinentes d11 pour l'obtention de potentiels d'action triés d12.

Différentes stratégies peuvent être utilisées à chacune de ces trois étapes. Lors de la première étape st10, la détection des potentiels d'action est généralement réalisée par une détection de franchissement de seuil en amplitude ou en énergie du signal brut. Lorsqu'un potentiel d'action est détecté, sa forme d'onde est enregistrée. La deuxième étape st11 d'extraction de caractéristiques pertinentes vise à construire l'espace des caractéristiques de formes d'ondes avant la troisième étape st12 de regroupement. Des caractéristiques prédéfinies telles que l'amplitude, la largeur, l'aire, les coefficients d'ondelettes ou la forme d'onde complète sur une certaine fenêtre de temps prédéfinie peuvent être utilisées comme caractéristiques pertinentes. La deuxième étape st11 peut également comprendre une sous-étape de réduction de la dimensionnalité de l'espace des caractéristiques. Pour cela, une approche classique est de réaliser une analyse en composantes principales PCA (de l'anglais « principal component analysis ») des matrices de caractéristiques, comme celles des formes d'ondes. La troisième étape st12 consiste alors à trier les potentiels d'action en les regroupant à partir des caractéristiques extraites et, le cas échéant, réduites. Il existe de nombreux algorithmes de regroupement, parmi lesquels l'algorithme k-moyennes (« k-means algorithm » en anglais) ou l'algorithme espérance-maximisation (« expectation-maximization algorithm » en anglais). Toutefois, ces méthodes de regroupement nécessitent généralement de connaître le nombre de groupes à réaliser. La supervision d'un opérateur est alors nécessaire afin de fournir cette donnée d'entrée.

Zhang et al. ont récemment proposé, dans leur article « A neuromorphic neural spike clustering processor for deep-brain sensing and stimulation systems » (ISLPED, 2015), un deuxième procédé 2 de tri de potentiels d'action au moyen d'un réseau de neurones artificiels implémentant des règles de plasticité fonction du temps d'occurrence des impulsions, ou règles STDP (de l'anglais « spike-timing-dependent plasticity »). La figure 1b montre un diagramme des étapes du deuxième procédé 2 selon l'art antérieur. Après la première étape st10 de détection des potentiels d'action et d'enregistrement des formes d'onde des potentiels d'action détectés d10, le deuxième procédé 2 comprend une deuxième étape st20 selon laquelle les formes d'onde d10 sont encodées sous forme d'impulsions d20, puis une troisième étape st21 selon laquelle les impulsions d20 sont injectées dans un réseau de neurones artificiels pour l'obtention de potentiels d'action triés d21. Une étape st22 de contrôle de la qualité des résultats obtenus par le réseau de neurones artificiels est prévue. Grâce aux règles STDP, le réseau de neurones artificiels apprend alors à reconnaître les différentes formes d'ondes des potentiels d'action. La première étape st10 de détection des potentiels d'action et d'enregistrement des formes d'onde des potentiels d'action détectés implique toutefois un prétraitement et un stockage des données en dehors du processeur neuromorphique implémentant le réseau de neurones artificiels. L'art antérieur pertinent dans le domaine de la présente invention inclut WO 2014/025765 A2, WO 2015/1834328 A1, US 2009/0216091, et "A Neuromorphic Event-Based Neural Recording System for Smart Brain-Machine-Interfaces" de F. Corradi et G. Indiveri, IEEE Transactions on Biomedical Circuits and Systems, Vol. 9 Nr. 5, October 2015., pp. 699 - 709.

On souhaite pouvoir trier en temps réel, à chaque instant, les potentiels d'action enregistrés par une pluralité de microélectrodes par un procédé de tri entièrement automatique, non supervisé et pouvant être entièrement implémenté sur un circuit neuromorphique.

## RESUME DE L'INVENTION

L'invention offre une solution aux problèmes évoqués précédemment, en permettant de trier des potentiels d'action en temps réel et de manière non supervisée, c'est-à-dire sans retour d'information fonction du résultat d'une couche de sortie, autrement dit sans rétroaction ou contreréaction, comme dans le cas d'apprentissage par renforcement. L'invention est définie aux revendications indépendantes 1 et 15.

Un aspect de l'invention concerne un procédé de tri non supervisé, en temps réel, de potentiels d'action d'une pluralité de neurones biologiques au moyen d'un réseau de neurones artificiels comportant une couche d'entrée, une couche intermédiaire et une couche de sortie, chaque neurone artificiel de la couche d'entrée étant connecté à une pluralité de neurones artificiels de la couche intermédiaire par une pluralité de premières synapses excitatrices et chaque neurone artificiel de la couche intermédiaire étant connecté à une pluralité de neurones artificiels de la couche de sortie par une pluralité de deuxièmes synapses excitatrices, au moins une première ou deuxième synapse excitatrice connectant un premier neurone artificiel à un deuxième neurone artificiel et ayant un poids qui est modifié en fonction des instants auxquels se produisent des impulsions présynaptiques émises par le premier neurone artificiel et des impulsions postsynaptiques émises par le deuxième neurone artificiel, procédé selon lequel :

- la couche d'entrée reçoit un signal électrique mesurant une activité électrique d'une pluralité de neurones biologiques, le signal électrique présentant une amplitude variable en fonction de potentiels d'action émis par la pluralité de neurones biologiques au cours du temps ;
- la couche d'entrée convertit l'amplitude du signal électrique en un train de premières impulsions ;
- la couche d'entrée transmet le train de premières impulsions à la couche intermédiaire ;
- la couche intermédiaire convertit le train de premières impulsions en un train de deuxièmes impulsions ;
- la couche intermédiaire transmet le train de deuxièmes impulsions à la couche de sortie ;
- en fonction du train de deuxièmes impulsions, la couche de sortie trie chaque occurrence de chaque type de potentiel d'action présente dans le signal électrique.

La propriété est exploitée selon laquelle la présence d'un type donné de potentiel d'action se traduit par une variation donnée d'amplitude dans le signal électrique. Il est ainsi possible de discriminer chaque type de potentiel d'action. Grâce à l'invention, la couche d'entrée acquiert directement le signal électrique mesurant l'activité électrique d'une pluralité de neurones biologiques. En s'affranchissant de l'étape de détection et d'encodage des procédés de l'art antérieur, le procédé de tri potentiels d'action selon un aspect de l'invention peut avantageusement être entièrement implémenté sur un circuit neuromorphique, sans nécessiter de prétraitement et/ou de stockage externe à ce circuit neuromorphique.

Dans le présent document, on entend par « la couche de sortie trie chaque occurrence de chaque type de potentiel d'action présente dans le signal électrique » le fait que, pour chaque type de potentiel d'action, la couche de sortie réalise un acte qui est propre audit type de potentiel d'action et qui est identique à chaque occurrence dudit type de potentiel d'action.

Outre les caractéristiques qui viennent d'être évoquées dans le paragraphe précédent, le procédé de tri selon un aspect de l'invention peut présenter une ou plusieurs caractéristiques complémentaires parmi les suivantes, considérées individuellement ou selon toutes les combinaisons techniquement possibles.

En fonction du train de deuxièmes impulsions la couche de sortie trie chaque occurrence de chaque type de potentiel d'action présente dans le signal électrique en activant au plus un unique groupe de neurones artificiels de la couche de sortie pour chaque occurrence d'un potentiel d'action dans le signal électrique, chaque groupe de neurones artificiels de la couche de sortie comportant au moins un neurone artificiel de la couche de sortie et étant associé à un unique type de potentiel d'action et chaque type de potentiel d'action étant associé à un unique groupe de neurones artificiels de la couche de sortie. Chaque groupe de neurones artificiels de la couche de sortie comporte préférentiellement un unique neurone artificiel de la couche de sortie. Alternativement, chaque groupe de neurones artificiels de la couche de sortie peut comporter plusieurs neurones artificiels de la couche de sortie, par exemple afin de permettre une redondance.

La couche d'entrée est une matrice de neurones artificiels ayant :

- Na lignes, le nombre Na de lignes étant choisi en fonction d'une plage de valeurs dans laquelle l'amplitude du signal électrique est variable, de manière que les neurones artificiels d'une même ligne s'activent pour une même valeur d'amplitude du signal électrique, et
- Nt colonnes, le nombre Nt de colonnes étant choisi en fonction d'une plage temporelle d'observation du signal électrique, de manière que les neurones artificiels d'une même colonne reçoivent le signal électrique à un même instant donné ;

et à chaque instant $t_n = n*dt$, avec n un entier naturel et dt un pas temporel :

- une première colonne $C_0$ de neurones artificiels reçoit le signal électrique, et
- en fonction de la valeur d'amplitude du signal électrique reçu, au moins un neurone artificiel de la première colonne $C_0$ émet une impulsion qui est transmise à la couche intermédiaire.

Le procédé de tri de potentiels d'action selon un aspect de l'invention réalise un stockage d'information uniquement sur une durée correspondant à la plage temporelle d'observation du signal électrique. La plage temporelle d'observation du signal électrique est typiquement de l'ordre de la durée d'un potentiel d'action. La plage temporelle d'observation du signal électrique est ainsi généralement comprise entre 0,1 ms et 5 ms, et préférentiellement comprise entre 0,5 ms et 1 ms.

Une marge est définie autour de chaque valeur d'amplitude du signal électrique reçu de manière que, à chaque instant $t_n = n*dt$ :

- la première colonne $C_0$ de neurones artificiels reçoit le signal électrique, et
- en fonction de la valeur d'amplitude du signal électrique reçu et de la marge définie autour de ladite valeur d'amplitude, au moins deux neurones artificiels de la première colonne $C_0$ émettent chacun une impulsion, chaque impulsion étant transmise à la couche intermédiaire.

Grâce à cette marge, on définit avantageusement un intervalle de sensibilité pour chaque neurone artificiel de la couche d'entrée. L'union des intervalles de sensibilité de tous les neurones artificiels de la couche d'entrée couvre typiquement la plage de valeurs dans laquelle l'amplitude du signal est variable. Il existe un recouvrement entre les intervalles de sensibilité de manière que pour chaque valeur donnée d'amplitude du signal électrique reçu, plusieurs neurones artificiels de la couche d'entrée réagissent en émettant chacun une impulsion. On augmente ainsi la robustesse du procédé selon un aspect de l'invention.

Le nombre Nt de colonnes de la couche d'entrée étant strictement supérieur à 1, à chaque instant $t_k = t_n + k*dt'$ et pour chaque colonne $C_k$ de neurones artificiels, avec dt' un deuxième pas temporel et k un entier naturel tel que $1 \leq k \leq Nt-1$, chaque neurone artificiel de même ligne qu'un neurone artificiel de la première colonne $C_0$ ayant émis une impulsion à l'instant $t_n$, émet à son tour une impulsion qui est transmise à la couche intermédiaire.

On permet ainsi que le signal électrique soit reçu uniquement par les neurones artificiels de la première colonne $C_0$, et pas nécessairement par l'ensemble des neurones artificiels de la couche d'entrée. L'information de variation d'amplitude du signal électrique, initialement reçue par la première colonne $C_0$ de neurones artificiels, peut être transmise de proche en proche aux autres colonnes de neurones artificiels de la couche d'entrée. Alternativement, la première colonne $C_0$ de neurones artificiels transformant l'information de variation d'amplitude du signal électrique qu'elle reçoit en une information d'émission d'impulsion par une ou plusieurs lignes de neurones artificiels, cette information d'émission d'impulsion peut être transmise de proche en proche aux autres colonnes de la couche d'entrée. Le deuxième pas temporel dt' peut être identique au pas temporel dt ou distinct du pas temporel dt.

Chaque neurone artificiel de la couche d'entrée est connecté à une pluralité de neurones artificiels de la couche intermédiaire par une pluralité de premières synapses excitatrices de manière qu'une impulsion émise par ledit neurone artificiel de la couche d'entrée est transmise à la pluralité de neurones artificiels de la couche intermédiaire par la pluralité de premières synapses excitatrices. Chaque neurone artificiel de la couche intermédiaire est connecté à une pluralité de neurones artificiels de la couche de sortie par une pluralité de deuxièmes synapses excitatrices de manière qu'une impulsion émise par ledit neurone artificiel de la couche intermédiaire est transmise à la pluralité de neurones artificiels de la couche de sortie par la pluralité de deuxièmes synapses excitatrices. Chaque synapse excitatrice présente un poids, chaque neurone artificiel de la couche intermédiaire et chaque neurone artificiel de la couche de sortie présentent un potentiel ayant initialement une valeur dite « de repos ».

Lorsqu'une synapse excitatrice transmet une impulsion à un neurone artificiel de la couche intermédiaire ou de la couche de sortie, le potentiel dudit neurone artificiel augmente proportionnellement au poids de ladite première synapse excitatrice. Lorsque le potentiel d'un neurone artificiel de la couche intermédiaire ou de la couche de sortie dépasse une valeur de seuil, ledit neurone artificiel émet une impulsion et son potentiel est abaissé à une valeur de réinitialisation ou alternativement ledit neurone artificiel est empêché de réémettre une impulsion pendant une période réfractaire. En l'absence d'impulsion en entrée d'un neurone artificiel de la couche intermédiaire ou de la couche de sortie et lorsque ce neurone n'émet lui-même pas d'impulsion, le potentiel dudit neurone revient à sa valeur de repos dans un temps caractéristique $T_m$.

Ce mécanisme permet d'éviter que les neurones artificiels émettent des impulsions de manière non spécifique.

La manière dont le potentiel de chaque neurone artificiel augmente ou revient à sa valeur de repos est typiquement déterminée par un modèle « intègre et décharge avec fuites » LIF (de l'anglais « leaky-integrate-and-fire »). Le poids d'une synapse est également appelé « poids synaptique ». Le potentiel d'un neurone artificiel est également appelé « potentiel de membrane » ou « potentiel transmembranaire ».

Le poids de chaque synapse excitatrice est modifié en fonction d'une fenêtre temporelle prédéfinie :

- pour une synapse excitatrice donnée, lorsque la fenêtre temporelle prédéfinie comporte une impulsion présynaptique et une impulsion postsynaptique, le poids de cette synapse excitatrice est augmenté ;
- pour une synapse excitatrice donnée, lorsque la fenêtre temporelle prédéfinie comporte une impulsion postsynaptique seule, le poids de cette synapse excitatrice est diminué ;
- pour une synapse excitatrice donnée, lorsque la fenêtre temporelle prédéfinie comporte une impulsion présynaptique seule, le poids de cette synapse excitatrice demeure inchangé.

Pour une synapse excitatrice donnée connectant un premier neurone artificiel à un deuxième neurone artificiel, on entend par « impulsion présynaptique » une impulsion émise par le premier neurone artificiel et transmise par ladite synapse excitatrice au deuxième neurone artificiel et on entend par « impulsion postsynaptique une impulsion émise par le deuxième neurone artificiel.

Ce mécanisme contribue à l'apprentissage, par le réseau de neurones artificiels, de chaque forme d'onde de potentiel d'action.

Chaque neurone artificiel de la couche d'entrée est connecté à une pluralité de neurones artificiels de la couche intermédiaire par une pluralité de premières synapses excitatrices de manière qu'une impulsion émise par un neurone artificiel de la couche d'entrée est transmise à la pluralité de neurones artificiels de la couche intermédiaire. Pour une première synapse excitatrice donnée connectant un neurone artificiel de la couche d'entrée à un neurone artificiel de la couche intermédiaire, on entend par « neurone artificiel présynaptique » le neurone artificiel de la couche d'entrée et on entend par « neurone artificiel postsynaptique » le neurone artificiel de la couche intermédiaire.

Selon un premier mode de réalisation, chaque première synapse excitatrice présente un poids affecté d'un coefficient synaptique $P_{rel}$ tel que $0 \leq P_{rel} \leq 1$, le coefficient synaptique $P_{rel}$ diminuant lorsque le neurone artificiel présynaptique émet une impulsion, et le coefficient synaptique $P_{rel}$ augmentant vers 1 sinon avec un temps caractéristique $T_{stp}$. Chaque neurone artificiel postsynaptique recevant une impulsion via une première synapse excitatrice est excité proportionnellement au produit du coefficient synaptique $P_{rel}$ et du poids de ladite première synapse excitatrice. Cette caractéristique, dite de « plasticité à court terme » entre la couche d'entrée et la couche intermédiaire, permet avantageusement de contribuer à filtrer le bruit du signal électrique reçu par la couche d'entrée afin que les neurones artificiels de la couche intermédiaire ne soient pas activés par du bruit, et donc que les neurones artificiels de la couche de sortie ne soient pas activés par du bruit.

Alternativement à la caractéristique de plasticité à court terme entre la couche d'entrée et la couche intermédiaire selon le premier mode de réalisation, une caractéristique de plasticité à court terme entre la couche intermédiaire et la couche de sortie peut être prévue afin que les neurones artificiels de la couche de sortie ne soient pas activés par du bruit. Chaque neurone artificiel de la couche intermédiaire est connecté à une pluralité de neurones artificiels de la couche de sortie par une pluralité de deuxièmes synapses excitatrices de manière qu'une impulsion émise par un neurone artificiel de la couche intermédiaire est transmise à la pluralité de neurones artificiels de la couche de sortie. Pour une deuxième synapse excitatrice donnée connectant un neurone artificiel de la couche intermédiaire à un neurone artificiel de la couche de sortie, on entend par « neurone artificiel présynaptique » le neurone artificiel de la couche intermédiaire et on entend par « neurone artificiel postsynaptique » le neurone artificiel de la couche de sortie. Chaque deuxième synapse excitatrice présente un poids affecté d'un coefficient synaptique $P_{rel}$ tel que $0 \leq P_{rel} \leq 1$, le coefficient synaptique $P_{rel}$ diminuant lorsque le neurone artificiel présynaptique émet une impulsion, et le coefficient synaptique $P_{rel}$ augmentant vers 1 sinon avec un temps caractéristique $T_{stp}$. Chaque neurone artificiel postsynaptique recevant une impulsion via une deuxième synapse excitatrice est excité proportionnellement au produit du coefficient synaptique $P_{rel}$ et du poids de ladite deuxième synapse excitatrice.

Il est possible de prévoir à la fois la caractéristique de plasticité à court terme entre la couche d'entrée et la couche intermédiaire, et la caractéristique de plasticité à court terme entre la couche intermédiaire et la couche de sortie.

Selon un deuxième mode de réalisation, le réseau de neurones artificiels comporte également au moins un neurone artificiel de détection, chaque neurone artificiel de la couche d'entrée étant connecté au neurone artificiel de détection par une première synapse excitatrice de détection, et le neurone artificiel de détection étant connecté à une pluralité de neurones artificiels de la couche intermédiaire par une pluralité de deuxièmes synapses excitatrices de détection. Selon le deuxième mode de réalisation, chaque première synapse excitatrice de détection présente un poids affecté d'un coefficient synaptique $P_{rel}$ tel que $0 \leq P_{rel} \leq 1$, le coefficient synaptique $P_{rel}$ diminuant lorsque le neurone artificiel présynaptique émet une impulsion et le coefficient synaptique $P_{rel}$ augmentant vers 1 sinon avec un temps caractéristique $T_{stp}$, et le neurone artificiel de détection recevant une impulsion via une première synapse excitatrice de détection est excité proportionnellement au produit du coefficient synaptique $P_{rel}$ et du poids de ladite première synapse excitatrice de détection. Selon le deuxième mode de réalisation, on implémente ainsi une règle de plasticité à court terme uniquement entre les neurones artificiels de la couche d'entrée et le neurone artificiel de détection, et pas entre les neurones artificiels de la couche d'entrée et les neurones artificiels de la couche intermédiaire. On sépare ainsi la fonction de détection binaire, c'est-à-dire « présence » ou « absence », des potentiels d'action d'une part, et la fonction de discrimination des potentiels d'action présents d'autre part : le neurone artificiel de détection assure la fonction de détection binaire grâce à la règle de plasticité à court terme tandis que les neurones artificiels de la couche intermédiaire assurent la fonction de discrimination grâce à une règle STDP. Le neurone artificiel de détection émet des impulsions lorsqu'il détecte la présence d'un potentiel d'action dans le train de premières impulsions émis par les neurones artificiels de la couche d'entrée et transmis via les premières synapses excitatrices de détection, et n'émet pas d'impulsion sinon. Les impulsions émises par le neurone artificiel de détection sont transmises aux neurones artificiels de la couche intermédiaire via les deuxièmes synapses excitatrices de détection. Ainsi, en fonction des impulsions qu'il émet, le neurone artificiel de détection module l'activité des neurones artificiels de la couche intermédiaire.

Selon un perfectionnement du deuxième mode de réalisation, le réseau de neurones artificiels comporte une synapse excitatrice récurrente, du neurone artificiel de détection vers lui-même. Ainsi, une fois que le seuil d'émission d'impulsion du neurone artificiel de détection est franchi, le neurone artificiel de détection reçoit une excitation supplémentaire par la synapse excitatrice récurrente. L'excitation provenant des neurones artificiels de la couche d'entrée doit donc descendre en-dessous d'un seuil inférieur au précédent seuil d'émission pour que le neurone artificiel de détection cesse d'émettre des impulsions, selon un mécanisme d'hystérésis. Cela permet d'assurer une continuité dans la détection, même dans le cas où la forme du potentiel d'action passe provisoirement par des valeurs proches de zéro. La fonction de détection est donc améliorée.

Selon un perfectionnement du premier ou du deuxième mode de réalisation, un neurone artificiel de la couche intermédiaire ou de la couche de sortie peut recevoir une impulsion d'un neurone artificiel de la couche précédente à la fois via une synapse excitatrice et via une synapse inhibitrice, chaque synapse obéissant à une règle STDP. La règle STDP de la synapse excitatrice est inversée par rapport à la règle STDP de la synapse inhibitrice, de manière que lorsque le poids de la synapse excitatrice augmente, celui de la synapse inhibitrice diminue, et inversement lorsque le poids de la synapse excitatrice diminue, celui de la synapse inhibitrice augmente. Ainsi, la somme des poids de la synapse excitatrice et de la synapse inhibitrice ne s'annule pas - sauf si chacun des deux poids est nul, car lorsque le poids de la synapse excitatrice est élevé, celui de la synapse inhibitrice est faible et inversement. Utiliser des contributions négatives par le biais de synapses inhibitrices, en plus des contributions positives des synapses excitatrices permet d'améliorer la reconnaissance des différents motifs de potentiels d'action.

Selon un autre perfectionnement du premier ou du deuxième mode de réalisation, un neurone artificiel de la couche intermédiaire ou de la couche de sortie peut recevoir une même impulsion d'un neurone artificiel de la couche précédente via une pluralité de synapses excitatrices ayant chacune un délai de transmission différent et/ou via une pluralité de synapses inhibitrices ayant chacune un délai de transmission différent, chaque synapse obéissant à une loi STDP. Par exemple, un neurone artificiel peut ainsi recevoir simultanément :

- une impulsion provenant d'un premier neurone artificiel de la couche précédente via une synapse ayant un délai court, et
- une impulsion provenant d'un deuxième neurone artificiel de la couche précédente via une synapse ayant un délai long.

Dans cet exemple, on a alors une information sur le fait que le deuxième neurone artificiel a émis une impulsion avant le premier neurone artificiel. D'une manière générale, ce perfectionnement permet à une couche de neurones artificiels d'avoir une information sur les instants relatifs d'émission d'impulsion par les neurones artificiels de la couche précédente. On améliore ainsi la précision du procédé de tri selon un aspect de l'invention, en lui permettant de différencier un plus grand nombre de motifs.

Selon un perfectionnement du deuxième mode de réalisation, le neurone artificiel de détection est connecté à une pluralité de neurones artificiels de la couche de sortie par une pluralité de deuxièmes synapses inhibitrices de détection : les impulsions du neurone artificiel de détection, transmises aux neurones artificiels de la couche de sortie via la pluralité de deuxièmes synapses inhibitrices de détection, forcent les neurones artificiels de la couche de sortie à attendre la fin d'un potentiel d'action avant d'émettre une impulsion. On contribue ainsi à contrôler l'instant d'émission d'une impulsion par les neurones artificiels de la couche de sortie. En particulier, on contribue à éviter qu'un neurone artificiel de la couche de sortie émette une impulsion avant la fin d'un potentiel d'action présent dans le signal. En effet, si un neurone artificiel de la couche de sortie émet une impulsion avant la fin d'un potentiel d'action, il risque de manquer des informations importantes pour trier le potentiel d'action en question, comme c'est par exemple le cas lorsque deux types de potentiels d'action commencent de la même manière mais finissent différemment. Selon ce perfectionnement, les neurones artificiels de la couche de sortie sont inhibés par le neurone artificiel de détection pendant toute la durée d'un potentiel d'action. On s'assure donc que les neurones artificiels de sortie puissent être excités après la fin du potentiel d'action, par exemple en introduisant un délai de transmission dans les connexions synaptiques entre la couche intermédiaire et la couche de sortie. En effet, comme les neurones artificiels de la couche de sortie sont inhibés pendant la durée d'un potentiel d'action, alors que les neurones artificiels de la couche intermédiaire n'émettent des impulsions que pendant la durée d'un potentiel d'action, si on ne s'assure pas que les neurones artificiels de la couche de sortie puissent être excités après la fin du potentiel d'action, ils ne seront a priori jamais excités.

Chaque neurone artificiel de la couche intermédiaire est connecté à l'ensemble des autres neurones artificiels de la couche intermédiaire par une pluralité de premières synapses inhibitrices de manière qu'une impulsion émise par ledit neurone artificiel de la couche intermédiaire est transmise à l'ensemble des autres neurones artificiels de la couche intermédiaire par la pluralité de premières synapses inhibitrices. Chaque neurone artificiel de la couche de sortie est connecté à l'ensemble des autres neurones artificiels de la couche de sortie par une pluralité de deuxièmes synapses inhibitrices de manière qu'une impulsion émise par ledit neurone artificiel de la couche de sortie est transmise à l'ensemble des autres neurones artificiels de la couche de sortie par la pluralité de deuxièmes synapses inhibitrices. Selon une première alternative, lorsqu'une synapse inhibitrice transmet une impulsion à un neurone artificiel de la couche intermédiaire ou de la couche de sortie, ledit neurone artificiel est empêché d'émettre une impulsion pendant une durée prédéfinie dite « période d'inhibition ». Selon une deuxième alternative, lorsqu'une synapse inhibitrice transmet une impulsion à un neurone artificiel de la couche intermédiaire ou de la couche de sortie, le potentiel dudit neurone artificiel est diminué proportionnellement au poids de ladite synapse inhibitrice. L'utilisation d'une période d'inhibition, dans la première alternative, permet de maîtriser le temps entre deux impulsions successives plus précisément que dans la deuxième alternative. La diminution de potentiel utilisée dans la deuxième alternative permet une plus grande souplesse : par exemple, si deux potentiels d'action parviennent simultanément ou presque, la couche de sortie pourra émettre deux impulsions correspondant à ces potentiels d'action malgré l'inhibition. Selon une troisième alternative combinant les première et deuxième alternatives, lorsqu'une synapse inhibitrice transmet une impulsion à un neurone artificiel de la couche intermédiaire ou de la couche de sortie, ledit neurone artificiel est empêché d'émettre une impulsion pendant une durée prédéfinie dite « période d'inhibition » et le potentiel dudit neurone artificiel est diminué proportionnellement au poids de ladite synapse inhibitrice.

Ce mécanisme de type « le gagnant emporte tout » (« winner takes all » en anglais) contribue avantageusement à empêcher que plusieurs neurones artificiels apprennent la même forme d'onde de potentiel d'action. Dans le cas où la couche de sortie active, pour chaque occurrence d'un potentiel d'action dans le signal électrique, un unique groupe de neurones artificiels de la couche de sortie comportant plusieurs neurones artificiels de la couche de sortie, il n'y a avantageusement pas d'inhibition latérale entre les neurones artificiels d'un même groupe.

Une pluralité de deuxièmes synapses excitatrices connecte chaque neurone artificiel de la couche intermédiaire à une pluralité de neurones artificiels de la couche de sortie, chaque deuxième synapse excitatrice ayant un poids. Pour chaque deuxième synapse excitatrice, lorsqu'un neurone artificiel de la couche de sortie reçoit, dans une fenêtre temporelle prédéfinie, une impulsion transmise par ladite deuxième synapse excitatrice et une impulsion transmise par une deuxième synapse inhibitrice, alors le poids de ladite deuxième synapse excitatrice est diminué. Pour chaque deuxième synapse excitatrice, lorsqu'un neurone artificiel de la couche de sortie reçoit, dans la fenêtre temporelle prédéfinie, une impulsion transmise par ladite deuxième synapse excitatrice et aucune impulsion transmise par une deuxième synapse inhibitrice, alors le poids de ladite deuxième synapse excitatrice est augmenté.

Ce mécanisme, appelé ici plasticité induite par inhibition, contribue avantageusement à empêcher que plusieurs neurones artificiels apprennent la même forme d'onde de potentiel d'action et augmente donc la précision de l'apprentissage. Le mécanisme de plasticité induite par inhibition est avantageusement combiné avec le mécanisme de type « le gagnant emporte tout » précédemment décrit.

Chaque neurone artificiel de la couche de sortie présente un potentiel ; chaque neurone artificiel de la couche de sortie émet une impulsion lorsque son potentiel dépasse une valeur de seuil. La valeur de seuil de chaque neurone artificiel de la couche de sortie émettant une impulsion à un instant t est augmentée proportionnellement au nombre d'impulsions reçues par ledit neurone artificiel dans une fenêtre temporelle autour de l'instant t. La valeur de seuil Th de chaque neurone artificiel de la couche de sortie émettant une impulsion à un instant t1 est diminuée de manière que :

$$\mathrm{Th}(t2) = \alpha * \mathrm{Th}(t1)$$

avec Th(t1) la valeur de seuil à l'instant t1, Th(t2) la valeur de seuil à un instant t2 postérieur à t1 et $\alpha$ un nombre réel tel que $0 < \alpha < 1$.

Le mécanisme ci-dessus introduit une plasticité sur la valeur de seuil de chaque neurone artificiel de la couche de sortie, appelée plasticité intrinsèque (« intrinsic plasticity » en anglais). On augmente ainsi avantageusement la robustesse du procédé selon un aspect de l'invention à d'éventuelles variations, d'une forme d'onde à l'autre, du nombre d'impulsions transmises à la couche de sortie. Lors d'une période d'initialisation, ou période d'apprentissage, du procédé de tri de potentiels d'action selon un aspect de l'invention, et bien qu'un mécanisme de type « le gagnant emporte tout » soit implémenté, il peut arriver que plusieurs neurones artificiels de la couche de sortie émettent, les uns après les autres, des impulsions pour un même potentiel d'action si leur seuil est mal adapté. Le mécanisme de plasticité intrinsèque qui vient d'être présenté a l'avantage de ne pas dépendre de l'instant t précis d'émission de chaque impulsion par un neurone artificiel de la couche de sortie, grâce à la fenêtre temporelle qui encadre l'instant t. Cela permet d'améliorer l'apprentissage en contribuant à garantir qu'un potentiel d'action active un unique groupe de neurones de la couche de sortie.

Alternativement, un autre mécanisme de plasticité intrinsèque peut être utilisé. La valeur de seuil de chaque neurone artificiel de la couche de sortie émettant une impulsion à un instant t est diminuée proportionnellement au nombre d'impulsions reçues par ledit neurone artificiel dans une première fenêtre temporelle avant l'instant t. La valeur de seuil de chaque neurone artificiel de la couche de sortie émettant une impulsion à un instant t est augmentée proportionnellement au nombre d'impulsions reçues par ledit neurone artificiel dans une deuxième fenêtre temporelle après l'instant t.

Le mécanisme alternatif ci-dessus introduit également avantageusement une plasticité sur la valeur de seuil de chaque neurone artificiel de la couche de sortie afin d'augmenter la robustesse du procédé selon un aspect de l'invention à d'éventuelles variations, d'une forme d'onde à l'autre, du nombre d'impulsions transmises à la couche de sortie.

Lorsqu'on introduit une plasticité intrinsèque sur la valeur de seuil de chaque neurone artificiel de la couche de sortie, selon l'une ou l'autre des deux alternatives qui viennent d'être présentées, on implémente aussi, préférentiellement, le mécanisme de type « le gagnant emporte tout » et le mécanisme de plasticité induite par inhibition entre la couche intermédiaire et la couche de sortie. On contribue ainsi à garantir qu'un potentiel d'action donné n'est appris que par un seul neurone artificiel de la couche de sortie, malgré la variabilité introduite sur la valeur de seuil de chaque neurone artificiel de la couche de sortie.

Le réseau de neurones artificiels comporte la couche d'entrée, la couche intermédiaire, une deuxième couche intermédiaire et la couche de sortie, et :

- la couche d'entrée transmet le train de premières impulsions vers la couche intermédiaire et vers la deuxième couche intermédiaire ;
- la deuxième couche intermédiaire convertit le train de premières impulsions en un deuxième train de deuxièmes impulsions ;
- la deuxième couche intermédiaire transmet le deuxième train de deuxièmes impulsions vers la couche de sortie ;
- en fonction du train de deuxièmes impulsions et du deuxième train de deuxièmes impulsions, la couche de sortie trie chaque occurrence de chaque type de potentiel d'action présente dans le signal électrique.

Dans cette configuration, un double traitement du train de premières impulsions est réalisé parallèlement par les première et deuxième couches intermédiaires. Cette configuration permet avantageusement d'augmenter la robustesse du procédé selon un aspect de l'invention. Le poids de chaque synapse excitatrice connectant un neurone artificiel de la couche d'entrée à un neurone artificiel de la couche intermédiaire ou à un neurone artificiel de la deuxième couche intermédiaire est typiquement initialisé aléatoirement. En conséquence, le train de deuxièmes impulsions en sortie de la couche intermédiaire est a priori différent du deuxième train de deuxièmes impulsions en sortie de la deuxième couche intermédiaire. Chaque couche intermédiaire est donc susceptible d'avoir, dans certains cas, de moins bons résultats que l'autre. Il est en revanche peu probable que deux couches intermédiaires différentes commettent les mêmes erreurs. C'est pourquoi combiner deux couches intermédiaires permet d'augmenter les performances.

Le réseau de neurones artificiels comporte la couche d'entrée, la couche intermédiaire, une deuxième couche intermédiaire et la couche de sortie, et :

- une partie de la couche d'entrée transmet un deuxième train de premières impulsions vers la deuxième couche intermédiaire ;
- la deuxième couche intermédiaire convertit le deuxième train de premières impulsions en un deuxième train de deuxièmes impulsions ;
- la deuxième couche intermédiaire transmet le deuxième train de deuxièmes impulsions vers la couche de sortie ;
- en fonction du train de deuxièmes impulsions et du deuxième train de deuxièmes impulsions, la couche de sortie trie chaque occurrence de chaque type de potentiel d'action présente dans le signal électrique.

Dans cette configuration, la couche d'entrée transmet le train de premières impulsions vers la couche intermédiaire tandis que la partie de la couche d'entrée transmet le deuxième train de premières impulsions vers la deuxième couche intermédiaire : le deuxième train de premières impulsions est une partie du train de premières impulsions. Autrement dit, le deuxième train de premières impulsions est inclus dans le train de premières impulsions.

La couche d'entrée étant une matrice de neurones artificiels, la partie de la couche d'entrée peut comporter le même nombre de lignes que la couche d'entrée et un nombre de colonnes inférieur à celui de la couche d'entrée. Dans ce cas, la partie de la couche d'entrée permet de prendre spécifiquement en compte une petite plage temporelle d'observation du signal électrique au sein de la plage temporelle d'observation de la couche d'entrée. Le choix de la taille de la plage temporelle d'observation a une influence sur les résultats obtenus. Plus la plage temporelle d'observation est grande, moins on subit d'erreurs dues au bruit. Cependant, une plage temporelle d'observation trop grande, c'est-à-dire typiquement une plage temporelle d'observation plus grande que la durée d'un potentiel d'action, contient beaucoup de signal non significatif et ne permet pas d'obtenir un résultat pertinent. Choisir plusieurs plages temporelles d'observation de tailles différentes permet donc de tirer le meilleur parti possible de chaque taille, sans connaître précisément la taille idéale de plage temporelle d'observation - qui n'est pas forcément la même pour les différents potentiels d'action. Alternativement, la partie de la couche d'entrée peut comporter le même nombre de colonnes que la couche d'entrée et un nombre de lignes inférieur à celui de la couche d'entrée.

Alternativement, le réseau de neurones artificiels comporte la couche d'entrée, une deuxième couche d'entrée, la couche intermédiaire et la couche de sortie, et :

- la deuxième couche d'entrée reçoit un deuxième signal électrique mesurant une activité électrique d'une deuxième pluralité de neurones biologiques, le deuxième signal électrique présentant une amplitude variable en fonction de potentiels d'action émis par la deuxième pluralité de neurones biologiques au cours du temps ;

- la deuxième couche d'entrée convertit l'amplitude du deuxième signal électrique en un deuxième train de premières impulsions ;
- la deuxième couche d'entrée transmet le deuxième train de premières impulsions à la couche intermédiaire ;
- la couche intermédiaire convertit le train de premières impulsions et le deuxième train de premières impulsions en un train de deuxièmes impulsions.

Le deuxième signal électrique peut être différent du premier signal électrique ou identique au premier signal électrique. Lorsque le deuxième signal électrique est différent du premier signal électrique, le deuxième signal électrique peut par exemple être la dérivée du premier signal électrique, ou les premier et deuxième signaux électriques peuvent être issus de deux microélectrodes extracellulaires distinctes. Cette configuration présente un intérêt particulier lorsque plusieurs microélectrodes extracellulaires proches les unes des autres sont utilisées pour enregistrer l'activité électrique des mêmes neurones biologiques. Le premier signal électrique est par exemple enregistré par une première microélectrode extracellulaire et le deuxième signal électrique est par exemple enregistré par une deuxième microélectrode extracellulaire proche de la première électrode extracellulaire ; les première et deuxième microélectrodes extracellulaires enregistrant l'activité électrique de neurones biologiques communs.

Les neurones artificiels de la couche d'entrée ayant un certain intervalle de sensibilité, les neurones artificiels de la deuxième couche d'entrée ont avantageusement un autre intervalle de sensibilité, distinct de l'intervalle de sensibilité des neurones artificiels de la couche d'entrée. En effet, chaque intervalle de sensibilité est préférentiellement choisi en fonction du niveau de bruit. En particulier, chaque intervalle de sensibilité est préférentiellement choisi sensiblement égal à trois fois le niveau de bruit. Mais cela suppose de connaître a priori le niveau de bruit.

Le réseau de neurones artificiels comporte la couche d'entrée, une deuxième couche d'entrée, la couche intermédiaire, une deuxième couche intermédiaire et la couche de sortie, et :

- la deuxième couche d'entrée reçoit un deuxième signal électrique mesurant une activité électrique d'une deuxième pluralité de neurones biologiques, le deuxième signal électrique présentant une amplitude variable en fonction de potentiels d'action émis par la deuxième pluralité de neurones biologiques au cours du temps ;
- la deuxième couche d'entrée convertit l'amplitude du deuxième signal électrique en un deuxième train de premières impulsions ;
- la deuxième couche d'entrée transmet le deuxième train de premières impulsions à la deuxième couche intermédiaire ;
- la deuxième couche intermédiaire convertit le deuxième train de premières impulsions en un deuxième train de deuxièmes impulsions ;
- la deuxième couche intermédiaire transmet le deuxième train de deuxièmes impulsions à la couche de sortie ;
- en fonction du train de deuxièmes impulsions et du deuxième train de deuxièmes impulsions, la couche de sortie trie chaque occurrence de chaque type de potentiel d'action présente dans le signal électrique.

Le deuxième signal électrique peut être différent du premier signal électrique ou identique au premier signal électrique.

Par rapport à la configuration précédente où plusieurs couches d'entrée sont connectées à une seule couche intermédiaire, cette configuration selon laquelle chaque couche d'entrée est connectée à sa propre couche intermédiaire permet avantageusement que chaque couche intermédiaire ait moins de motifs, ou formes d'onde, à apprendre : on augmente ainsi la robustesse et les performances du réseau de neurones artificiels.

Un autre aspect de l'invention concerne un réseau de neurones artificiels pour la mise en oeuvre d'un procédé de tri non supervisé, en temps réel, de potentiels d'action d'une pluralité de neurones biologiques, le réseau de neurones artificiels comportant :

- une couche d'entrée pour la réception d'un signal électrique présentant une amplitude variable en fonction de potentiels d'action émis par la pluralité de neurones biologiques au cours du temps, la conversion du signal électrique en un train de premières impulsions et la transmission du train de premières impulsions à une couche intermédiaire ;
- ladite couche intermédiaire pour la conversion du train de premières impulsions en un train de deuxièmes impulsions et la transmission du train de deuxièmes impulsions à une couche de sortie ;
- ladite couche de sortie pour le tri, en fonction du train de deuxièmes impulsions, de chaque occurrence de chaque type de potentiel d'action présente dans le signal électrique ;

chaque neurone artificiel de la couche d'entrée étant connecté à une pluralité de neurones artificiels de la couche intermédiaire par une pluralité de premières synapses excitatrices et chaque neurone artificiel de la couche intermédiaire étant connecté à une pluralité de neurones artificiels de la couche de sortie par une pluralité de deuxièmes synapses excitatrices, chaque première ou deuxième synapse excitatrice connectant un premier neurone artificiel à un deuxième neurone artificiel et ayant un poids qui est modifié en fonction des instants auxquels se produisent des impulsions présynaptiques émises par le premier neurone artificiel et des impulsions postsynaptiques émises par le deuxième neurone artificiel.

L'invention et ses différentes applications seront mieux comprises à la lecture de la description qui suit et à l'examen des figures qui l'accompagnent.

## BREVE DESCRIPTION DES FIGURES

Les figures sont présentées à titre indicatif et nullement limitatif de l'invention.

- La figure 1a montre un diagramme des étapes d'un premier procédé de tri supervisé et en différé de potentiels d'action selon l'art antérieur.
- La figure 1b montre un diagramme des étapes d'un deuxième procédé de tri en différé de potentiels d'action selon l'art antérieur.
- La figure 2a montre un diagramme des étapes d'un procédé selon un aspect de l'invention de tri non supervisé et en temps réel de potentiels d'action d'une pluralité de neurones biologiques au moyen d'un réseau de neurones artificiels.
- La figure 2b montre schématiquement un premier réseau de neurones artificiels selon un premier mode de réalisation pour la mise en oeuvre du procédé de la figure 2a, le premier réseau de neurones artificiels selon le premier mode de réalisation comportant une couche d'entrée, une couche intermédiaire et une couche de sortie.
- La figure 2c montre schématiquement un premier réseau de neurones artificiels selon un deuxième mode de réalisation pour la mise en oeuvre du procédé de la figure 2a, le premier réseau de neurones artificiels selon le deuxième mode de réalisation comportant en outre un neurone artificiel de détection.
- La figure 3a montre schématiquement la couche d'entrée du premier réseau de neurones artificiels de la figure 2b recevant un signal électrique et convertissant l'amplitude du signal électrique en un train de premières impulsions.
- La figure 3b illustre schématiquement la définition d'une marge autour d'une valeur d'amplitude pour l'obtention d'un intervalle de sensibilité d'un neurone artificiel de la couche d'entrée du premier réseau de neurones artificiels de la figure 2b.
- La figure 3c montre schématiquement un chevauchement des intervalles de sensibilité des neurones artificiels de la couche d'entrée du premier réseau de neurones artificiels de la figure 2b.
- La figure 4a montre schématiquement une pluralité de premières synapses inhibitrices interconnectant les neurones artificiels de la couche intermédiaire du premier réseau de neurones artificiels de la figure 2b.
- La figure 4b montre schématiquement une pluralité de deuxièmes synapses inhibitrices interconnectant les neurones artificiels de la couche de sortie du premier réseau de neurones artificiels de la figure 2b.
- La figure 5 montre schématiquement un deuxième réseau de neurones artificiels pour la mise en oeuvre du procédé de la figure 2a.
- La figure 6 montre schématiquement un troisième réseau de neurones artificiels pour la mise en oeuvre du procédé de la figure 2a.
- La figure 7 montre schématiquement un quatrième réseau de neurones artificiels pour la mise en oeuvre du procédé de la figure 2a.
- La figure 8 montre schématiquement un cinquième réseau de neurones artificiels pour la mise en oeuvre du procédé de la figure 2a.

## DESCRIPTION DETAILLEE D'AU MOINS UN MODE DE REALISATION DE L'INVENTION

Sauf précision contraire, un même élément apparaissant sur des figures différentes présente une référence unique.

Dans la présente demande, on emploie indifféremment les termes « synapse » et « synapse artificielle ». Dans le cas d'une synapse connectant un premier neurone artificiel à un deuxième neurone artificiel :

- le premier neurone artificiel est aussi appelé « neurone présynaptique », vis-à-vis de ladite synapse, et
- le deuxième neurone artificiel est aussi appelé « neurone postsynaptique », vis-à-vis de ladite synapse.

Lorsque le premier neurone émet une impulsion qui est transmise au deuxième neurone artificiel, cette impulsion est aussi appelée « impulsion présynaptique », vis-à-vis du deuxième neurone artificiel. Lorsque le deuxième neurone artificiel émet une impulsion, cette impulsion est aussi appelée « impulsion postsynaptique », vis-à-vis du deuxième neurone artificiel.

Les figures 1a et 1b ont été décrites dans l'arrière-plan technologique de l'invention.

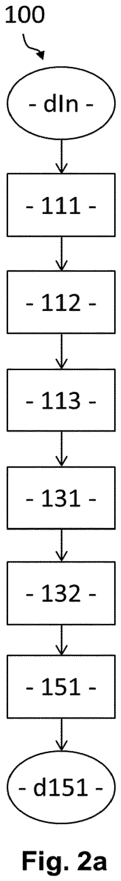

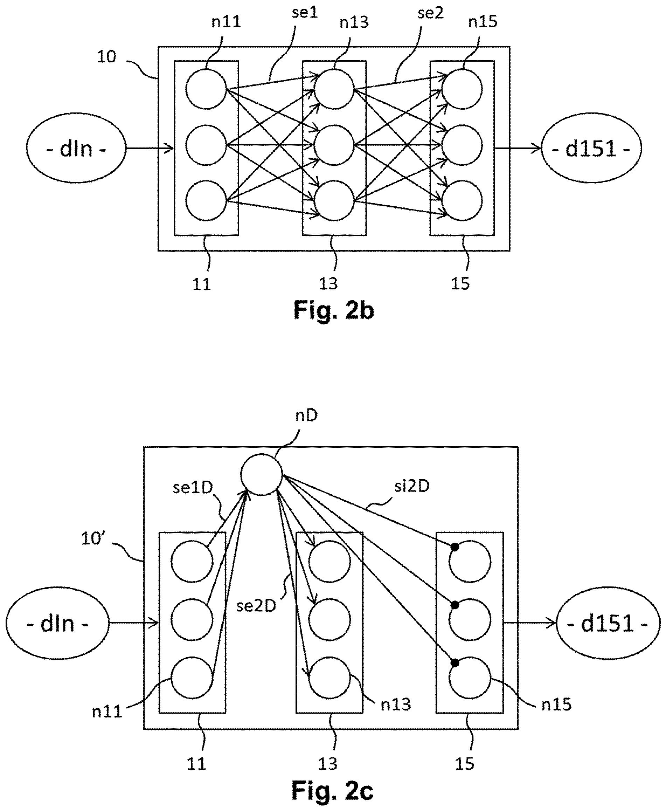

La figure 2a montre un diagramme des étapes d'un procédé 100 selon un aspect de l'invention de tri non supervisé et en temps réel de potentiels d'action d'une pluralité de neurones biologiques au moyen d'un réseau de neurones artificiels. La figure 2b montre schématiquement un premier réseau de neurones artificiels 10 selon un premier mode de réalisation, pour la mise en oeuvre du procédé 100. La figure 2c montre schématiquement un premier réseau de neurones artificiels 10' selon un deuxième mode de réalisation, pour la mise en oeuvre du procédé 100. Les figures 2a, 2b et 2c sont décrites conjointement.

Le premier réseau de neurones artificiels 10, 10' selon le premier ou le deuxième mode de réalisation comporte :

- une couche d'entrée 11 comportant une pluralité de neurones artificiels n11,
- une couche intermédiaire 13 comportant une pluralité de neurones artificiels n13 et
- une couche de sortie 15 comportant une pluralité de neurones artificiels n15.

Selon un mode de réalisation, dit « all-to-all » en anglais, une pluralité de premières synapses excitatrices se1 connecte chaque neurone artificiel de la couche d'entrée n11 à la pluralité de neurones artificiels de la couche intermédiaire n13. Une pluralité de deuxièmes synapses excitatrices se2 connecte chaque neurone artificiel de la couche intermédiaire n13 à la pluralité de neurones artificiels de la couche de sortie n15. Autrement dit :

- chaque neurone artificiel de la couche intermédiaire n13 reçoit une première synapse excitatrice se1 venant de chaque neurone artificiel de la couche d'entrée n11, et
- chaque neurone artificiel de la couche intermédiaire n13 envoie une deuxième synapse excitatrice se2 vers chaque neurone artificiel de la couche de sortie n15.

Alternativement à une connexion « all-to-all » entre les neurones artificiels n11 de la couche d'entrée et les neurones artificiels n13 de la couche intermédiaire d'une part, et entre les neurones artificiels n13 de la couche intermédiaire et les neurones artificiels n15 de la couche de sortie d'autre part, une ou plusieurs connexions partielles peuvent être envisagées. Par exemple, un ou plusieurs neurones artificiels n11 de la couche d'entrée peuvent n'être connectés qu'à une partie des neurones artificiels n13 de la couche intermédiaire. De même, un ou plusieurs neurones artificiels n13 de la couche intermédiaire peuvent n'être connectés qu'à une partie des neurones artificiels n15 de la couche de sortie.

Selon le mode de réalisation généralement décrit dans la présente demande, toutes les synapses excitatrices du premier réseau de neurones artificiels 10 vont dans la même direction (« feedforward excitation » en anglais), en l'occurrence : de la couche d'entrée 11 vers la couche de sortie 15. On peut alternativement utiliser un réseau de neurones artificiels, dit « récurrent », dans lequel certaines synapses excitatrices reviennent en arrière, de la couche de sortie vers la couche d'entrée.

Le premier réseau de neurones artificiels 10' selon le deuxième mode de réalisation, illustré à la figure 2c, comporte en outre un neurone artificiel de détection nD. Dans un souci de clarté, les première et deuxième synapses excitatrices se1, se2 ne sont pas représentées à la figure 2c. Chaque neurone artificiel n11 de la couche d'entrée est connecté au neurone artificiel de détection nD via une première synapse excitatrice de détection se1D, et le neurone artificiel de détection nD est connecté à chaque neurone artificiel n13 de la couche intermédiaire via une pluralité de deuxièmes synapses excitatrices de détection se2D. Alternativement, seulement une partie des neurones artificiels n11 de la couche d'entrée pourrait être connectée au neurone artificiel de détection nD, et/ou le neurone artificiel de détection nD pourrait n'être connecté qu'à une partie seulement des neurones artificiels n13 de la couche intermédiaire.

Selon un perfectionnement du premier réseau de neurones artificiels 10' selon le deuxième mode de réalisation, le neurone artificiel de détection nD est connecté à chaque neurone artificiel n15 de la couche de sortie par une deuxième synapse inhibitrice de détection si2D.

Selon une étape 111 du procédé 100 de tri non supervisé et en temps réel de potentiels d'action d'une pluralité de neurones biologiques, la couche d'entrée 11 reçoit un signal électrique dIn. Le signal électrique dIn mesure une activité électrique d'une pluralité de neurones biologiques et présente une amplitude variable en fonction de potentiels d'action émis par la pluralité de neurones biologiques au cours du temps.

Selon une étape 112 du procédé 100, la couche d'entrée 11 convertit ensuite l'amplitude du signal électrique dIn en un train de premières impulsions. Le train de premières impulsions comprend toutes les impulsions qui sont émises par les neurones artificiels de la couche d'entrée n11.

Après l'étape 112, le procédé 100 comporte une étape 113 selon laquelle la couche d'entrée 11 transmet le train de premières impulsions à la couche intermédiaire 13 via la pluralité de premières synapses excitatrices se1.

La couche intermédiaire 13 convertit alors le train de premières impulsions en un train de deuxièmes impulsions, selon une étape 131 du procédé 100, puis le train de deuxièmes impulsions est transmis à la couche de sortie 15 via la pluralité de deuxièmes synapses excitatrices se2 lors d'une étape 132 du procédé 100. Le train de deuxièmes impulsions comprend toutes les impulsions qui sont émises par les neurones artificiels de la couche intermédiaire n13.

Enfin, dans l'étape 151 du procédé 100, la couche de sortie 15 trie chaque occurrence de chaque type de potentiel d'action présente dans le signal électrique dln, en fonction du train de deuxièmes impulsions en activant au plus un unique groupe de neurones artificiels de la couche de sortie n15 pour chaque occurrence d'un potentiel d'action dans le signal électrique dln, chaque groupe de neurones artificiels de la couche de sortie n15 étant associé à un unique type de potentiel d'action et chaque type de potentiel d'action étant associé à un unique groupe de neurones artificiels de la couche de sortie. Un groupe de neurones artificiels peut comporter plusieurs neurones artificiels ou bien, préférentiellement, un unique neurone artificiel.

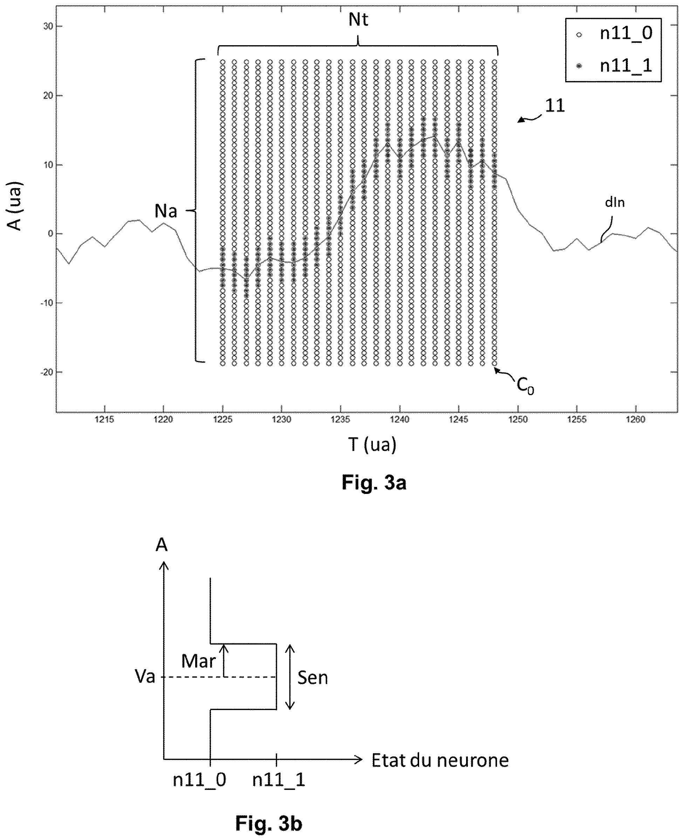

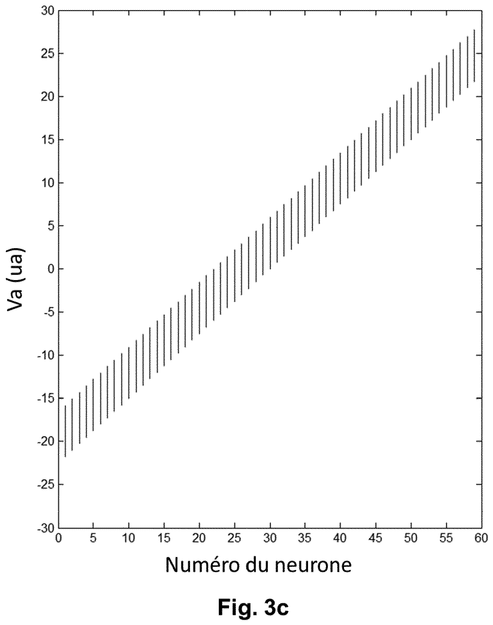

La figure 3a montre schématiquement la couche d'entrée 11 lorsqu'elle reçoit le signal électrique dln puis convertit l'amplitude du signal électrique dln en un train de premières impulsions. La figure 3b illustre schématiquement la définition d'une marge Mar autour d'une valeur d'amplitude Va pour l'obtention d'un intervalle de sensibilité Sen d'un neurone artificiel de la couche d'entrée n11. La figure 3c montre schématiquement un chevauchement des intervalles de sensibilité Sen des neurones artificiels de la couche d'entrée n11. Les figures 3a, 3b et 3c sont décrites conjointement.

La couche d'entrée 11 est typiquement une matrice de neurones artificiels n11 comportant Na lignes et Nt colonnes. Le nombre Nt de colonnes est typiquement choisi en fonction d'une plage temporelle d'observation du signal électrique dln, de manière que les neurones artificiels n11 d'une même colonne reçoivent le signal électrique à un même instant donné.

Le nombre Na de lignes est typiquement choisi en fonction d'une plage de valeurs dans laquelle l'amplitude du signal électrique est variable, de manière que :

- les neurones artificiels n11 d'une même ligne s'activent pour une même valeur d'amplitude du signal électrique dln, et
- les neurones artificiels de lignes distinctes s'activent pour des valeurs distinctes d'amplitude Va du signal électrique dln.

Chaque neurone artificiel n11 d'une même colonne est ainsi associé à une valeur distincte d'amplitude Va du signal électrique dln de manière que l'ensemble des neurones artificiels n11 d'une même colonne balaie la plage de valeurs dans laquelle l'amplitude du signal électrique dln est variable.

A chaque instant $t_n = n*dt$, avec n un entier naturel et dt un pas temporel, une première colonne $C_0$ de neurones artificiels n11 reçoit le signal électrique dln et en fonction de la valeur d'amplitude du signal électrique dln reçu, au moins un neurone artificiel n11 de la première colonne $C_0$ émet une impulsion qui est transmise à la couche intermédiaire 13. Sur la figure 3a, un neurone artificiel émettant une impulsion est référencé 11_1 et représenté par un cercle plein tandis qu'un neurone artificiel n'émettant pas d'impulsion est référencé 11_0 et représenté par un cercle vide.

On définit préférentiellement une marge Mar autour de chaque valeur d'amplitude Va du signal électrique dln reçu de manière que chaque neurone artificiel de la couche d'entrée n11 présente un intervalle de sensibilité Sen. Ainsi, un neurone artificiel de la couche d'entrée n11 émet une impulsion lorsque la valeur d'amplitude Va du signal électrique dln est dans l'intervalle de sensibilité dudit neurone artificiel n11. Le nombre Na de lignes et la taille des intervalles de sensibilité Sen sont préférentiellement choisis en fonction de la plage de valeurs dans laquelle l'amplitude du signal électrique est variable de manière que :

- l'ensemble des intervalles de sensibilité Sen balaie la plage de valeurs dans laquelle l'amplitude du signal électrique dln est variable, et
- les neurones artificiels n11 de lignes consécutives ont leurs intervalles de sensibilité Sen qui se chevauchent.

Grâce aux intervalles de sensibilité Sen et à leur chevauchement, à chaque instant $t_n = n*dt$ :

- la première colonne $C_0$ de neurones artificiels n11 reçoit un échantillon du signal électrique dln, et
- au moins deux neurones artificiels n11 de la première colonne $C_0$ émettent chacun une impulsion, chaque impulsion étant transmise à la couche intermédiaire 13.

Dans l'exemple particulier de la figure 3c, la marge Mar autour de chaque valeur d'amplitude Va est choisie égale à 1,5 fois le niveau de bruit et la largeur de l'intervalle de sensibilité Sen est donc égale à 3 fois le niveau de bruit. Ce choix assure une distribution de probabilité des impulsions permettant un bon apprentissage. Le chevauchement des intervalles de sensibilité Sen est ensuite paramétré afin que huit neurones artificiels n11 de la première colonne $C_0$ émettent une impulsion à chaque instant $t_n$.

Le nombre Nt de colonnes étant strictement supérieur à 1, à chaque instant $t_k = t_n + k*dt'$ et pour chaque colonne $C_k$ de neurones artificiels n11, avec dt' un deuxième pas temporel et k un entier naturel tel que $1 \leq k < (Nt - 1)$, chaque neurone artificiel n11 de même ligne qu'un neurone artificiel n11 de la première colonne $C_0$ ayant émis une

impulsion à l'instant $t_n$, émet à son tour une impulsion qui est transmise à la couche intermédiaire 13. Le nombre Nt de colonnes est préférentiellement choisi de sorte que la plage temporelle d'observation du signal électrique dln est légèrement inférieure à la durée minimum d'un potentiel d'action. Dans un exemple d'implémentation, la plage temporelle d'observation du signal électrique dln est choisie égale à 0,9 ms. Dans un exemple d'implémentation, le deuxième pas temporel dt' est égal au pas temporel dt.

Dans le mode de réalisation qui a été décrit jusqu'à présent, chaque neurone artificiel n11 de la couche d'entrée est sensible à une valeur d'amplitude à un instant donné. Alternativement, chaque neurone artificiel n11 de la couche d'entrée peut être sensible aux valeurs d'amplitude dans une fenêtre temporelle de sensibilité donnée. Dans ce cas, un neurone artificiel n11 de la couche d'entrée émet une impulsion si au moins une des valeurs d'amplitude reçues pendant sa fenêtre temporelle de sensibilité est dans son intervalle de sensibilité. Ce mode de réalisation alternatif peut apporter une plus grande robustesse au procédé 100 selon un aspect de l'invention, particulièrement lorsque le signal électrique associé à un potentiel d'action comporte de rapides variations d'amplitude, ce qui a pour conséquence que les valeurs d'amplitude échantillonnées dépendent fortement de la synchronisation entre ledit potentiel d'action et l'échantillonnage.

Le comportement des neurones artificiels de la couche intermédiaire n13 et des neurones artificiels de la couche de sortie n15 est typiquement défini par un modèle LIF (de l'anglais « Leaky-Integrate-and-Fire »). Selon ce modèle LIF, chaque neurone artificiel présente un potentiel, également appelé potentiel de membrane ou potentiel transmembranaire et chaque synapse présente un poids, également appelé poids synaptique. A chaque fois qu'un neurone artificiel reçoit une impulsion via une synapse, le potentiel de ce neurone artificiel est modifié proportionnellement au poids de cette synapse. Dans le cas d'une synapse excitatrice, le potentiel du neurone artificiel est augmenté proportionnellement au poids de cette synapse ; dans le cas d'une synapse inhibitrice, le potentiel du neurone artificiel est diminué proportionnellement au poids de cette synapse. Lorsque le potentiel d'un neurone artificiel atteint une certaine valeur de seuil Th, le neurone artificiel émet une impulsion et son potentiel est ramené à une valeur de réinitialisation $V_{RESET}$. La valeur de réinitialisation $V_{RESET}$ est notamment choisie en fonction de la valeur de seuil : l'objectif est que la valeur du potentiel d'un neurone artificiel ayant émis une impulsion soit ramenée à une valeur proche de la valeur du potentiel de repos, ou inférieure à la valeur du potentiel de repos.

Selon le modèle LIF, la valeur du potentiel de chaque neurone artificiel respecte l'équation suivante :

$$\frac{dV}{dt} = -\frac{1}{\tau_m} * V(t) + \sum_i \sum_s w_i(t_s)\delta(t - t_{i,s}) + \sum_f (V_{reset} - V(t_f))\delta(t - t_f)$$

$T_m$ est le temps caractéristique de membrane du neurone artificiel considéré ; i indexe chaque synapse entrante, de poids w, du neurone artificiel considéré ; s indexe les impulsions reçues par le neurone artificiel considéré de la part de chaque synapse i, avec leur temps d'arrivée $t_{i,s}$ ; f indexe les impulsions émises par le neurone artificiel considéré avec leur temps d'émission $t_f$.

Les valeurs du temps caractéristique $T_m$ et de la valeur de seuil Th sont choisies en fonction de la couche considérée. Le choix de la valeur de seuil dépend du nombre d'impulsions attendues pour la couche considérée.

Pour chaque neurone artificiel n13 de la couche intermédiaire :

- le temps caractéristique membranaire $T_m$ est préférentiellement compris dans l'intervalle [dt ; 6*dt] ; d'une manière générale, les neurones artificiels n13 de la couche intermédiaire apprenant des portions, à un instant donné, de forme d'onde des potentiels d'action, on choisit préférentiellement pour les neurones artificiels n13 de la couche intermédiaire un temps caractéristique $T_m$ du même ordre de grandeur que la période d'échantillonnage dt ;
- la valeur de seuil Th est choisie de manière à être légèrement supérieure au potentiel des neurones artificiels lorsque le signal électrique ne contient que du bruit ;
- la valeur de réinitialisation $V_{RESET}$ est préférentiellement comprise dans l'intervalle [-100*Th ; 0].

Selon un exemple particulier d'implémentation, pour chaque neurone artificiel n13 de la couche intermédiaire :

- le temps caractéristique membranaire $T_m$ est égal à 3*dt ;
- la valeur de seuil Th est égale à Nt*overlap*0,75 avec Nt le nombre de colonnes de la couche d'entrée et « overlap » le chevauchement des intervalles de sensibilité Sen des neurones artificiels de la couche d'entrée - qui est fixé à 8 dans l'exemple particulier de la figure 3c ;
- la valeur de réinitialisation $V_{RESET}$ est égale à -20*Th.

Pour chaque neurone artificiel n15 de la couche de sortie :

- le temps caractéristique membranaire $T_m$ est préférentiellement compris dans l'intervalle [1ms ; 4ms] ; d'une manière générale, le rôle de chaque neurone artificiel n15 de la couche de sortie étant d'apprendre l'intégralité d'un potentiel d'action, le temps caractéristique $T_m$ des neurones artificiels n15 de la couche de sortie est préférentiellement choisi de l'ordre de la durée d'un potentiel d'action ;
- la valeur de réinitialisation $V_{RESET}$ est préférentiellement comprise dans l'intervalle [-20*Th ; 0].

Selon un exemple particulier d'implémentation, pour chaque neurone artificiel n15 de la couche de sortie :

- le temps caractéristique membranaire $T_m$ est égal à 2,5 ms ;
- la valeur de réinitialisation $V_{RESET}$ est égale à -5*Th.

On prévoit avantageusement que chaque neurone artificiel de la couche intermédiaire, après avoir émis une impulsion, est empêché d'émettre une autre impulsion pendant une période réfractaire. Cette période réfractaire est typiquement de l'ordre de grandeur de la durée d'une période d'échantillonnage dt et préférentiellement comprise dans l'intervalle [dt ; 10*dt]. Dans l'exemple particulier d'implémentation, la période réfractaire est de 5*dt. De même, on prévoit avantageusement que chaque neurone artificiel de la couche de sortie, après avoir émis une impulsion, est empêché d'émettre une autre impulsion pendant une deuxième période réfractaire. Cette deuxième période réfractaire est typiquement de l'ordre de grandeur de la durée d'un potentiel d'action, préférentiellement comprise entre 0,5 ms et 10 ms et plus préférentiellement comprise entre 1 ms et 4 ms. Dans l'exemple particulier d'implémentation, la période réfractaire est de 2,5 ms. Le poids de chaque première synapse excitatrice se1 est préférentiellement normalisé : le poids de chaque première synapse excitatrice se1 appartient alors à l'intervalle [0 ; 1]. Le poids de chaque deuxième synapse excitatrice se2 est préférentiellement normalisé : le poids de chaque deuxième synapse excitatrice se2 appartient alors à l'intervalle [0 ; 1].

Chaque synapse excitatrice, c'est-à-dire chaque première synapse excitatrice se1 et chaque deuxième synapse excitatrice se2 dans le cas du réseau de neurones artificiels 10, respecte avantageusement une règle STDP qui modifie son poids en fonction des instants auxquels se produisent respectivement les impulsions présynaptiques et les impulsions postsynaptiques. On définit typiquement, pour chaque synapse excitatrice, une fenêtre temporelle de coïncidence $\Delta t$ entre impulsions présynaptique et postsynaptique :

$$\Delta t = \left[ -\tau_{stdp-} ; +\tau_{stdp+} \right]$$

où $T_{stdp-}$ est une durée mesurée en amont de chaque impulsion présynaptique reçue par ladite synapse excitatrice et $T_{stdp+}$ est une durée mesurée en aval de chaque impulsion présynaptique reçue par ladite synapse excitatrice.

Cette fenêtre temporelle de coïncidence est préférentiellement choisie en fonction du positionnement de chaque synapse excitatrice au sein du réseau de neurones artificiels. Pour chaque première synapse excitatrice si1 :

- $T_{stdp+}$ appartient préférentiellement à l'intervalle [0 ; 3*dt] et dans l'exemple particulier de réalisation, $T_{stdp+}$ est égal à 1*dt ;
- $T_{stdp-}$ appartient préférentiellement à l'intervalle [0 ; 3*dt] et dans l'exemple particulier de réalisation, $T_{stdp-}$ est égal à 0.

Pour chaque deuxième synapse excitatrice si2 :

- $T_{stdp+}$ appartient préférentiellement à l'intervalle [1 ms ; 3 ms] et dans l'exemple particulier de réalisation, $T_{stdp+}$ est égal à 1,5 ms ;
- $T_{stdp-}$ appartient préférentiellement à l'intervalle [1 ms ; 3 ms] et dans l'exemple particulier de réalisation, $T_{stdp-}$ est égal à 1,5 ms.

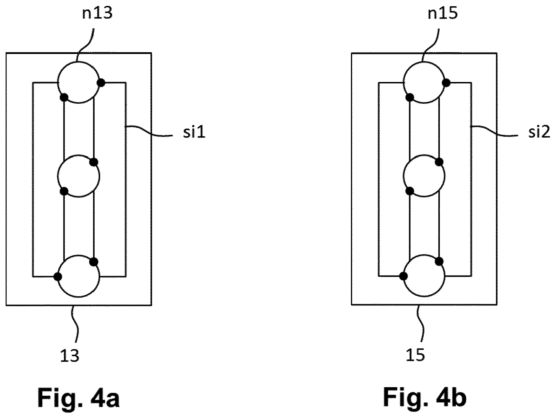

On utilise avantageusement un mécanisme de type « le gagnant emporte tout » (« winner takes all » en anglais) au sein de la couche intermédiaire 13 et au sein de la couche de sortie 15. Ce mécanisme est illustré aux figures 4a et 4b, qui sont décrites conjointement. Selon ce mécanisme, chaque neurone artificiel n13 de la couche intermédiaire est connecté à tous les autres neurones artificiels n13 de la couche intermédiaire par une pluralité de premières synapses inhibitrices si1. De même, chaque neurone artificiel n15 de la couche de sortie est connecté à tous les autres neurones artificiels n15 de la couche de sortie par une pluralité de deuxièmes synapses inhibitrices si2. Ainsi, un neurone artificiel émettant une impulsion à un instant donné inhibe latéralement les autres neurones de la même couche afin d'empêcher qu'ils émettent eux aussi une impulsion au même instant. Cette inhibition latérale contribue à ce que les neurones artificiels des couches intermédiaire et de sortie apprennent à émettre des impulsions pour des formes d'onde différentes en entrée. Il est en effet possible que plusieurs neurones artificiels d'une même couche aient tendance à apprendre les mêmes formes d'onde. Dans ce cas, l'inhibition latérale contraint ces neurones artificiels à ne pas émettre leurs impulsions exactement au même moment, donc à décaler leurs émissions en respectant un certain délai de latence. Le délai de latence est déterminé par le paramétrage de l'inhibition latérale : plus l'inhibition latérale est forte, plus le délai de latence est important. Ainsi, grâce à l'inhibition latérale, les neurones artificiels d'une même couche apprennent soit à émettre des impulsions pour des potentiels d'action différents en entrée, soit à émettre des impulsions pour des parties différentes d'un même potentiel d'action en entrée. Le poids de chaque première synapse inhibitrice si1 est préférentiellement compris dans l'intervalle [0,1*Th ; 0,5*Th], avec Th la valeur de seuil de chaque neurone artificiel n13 de la couche intermédiaire. Dans l'exemple particulier d'implémentation, le poids de chaque première synapse inhibitrice si1 est égal à 0,2*Th.

Le poids de chaque deuxième synapse inhibitrice si2 est préférentiellement compris dans l'intervalle [1*Th ; 10*Th], avec Th la valeur de seuil de chaque neurone artificiel n15 de la couche de sortie. Dans l'exemple particulier d'implémentation, le poids de chaque deuxième synapse inhibitrice si2 est égal à 10*Th.

Une règle STDP (de l'anglais « spike-timing-dependent plasticity ») peut également être implémentée pour chaque synapse inhibitrice, c'est-à-dire pour chaque première synapse inhibitrice si1 et pour chaque deuxième synapse inhibitrice si2 dans le cas du réseau de neurones artificiels 10, selon laquelle :

- le poids de chaque synapse inhibitrice augmente en cas de coïncidence d'une impulsion postsynaptique et d'une impulsion présynaptique dans une certaine fenêtre temporelle, et
- le poids de chaque synapse inhibitrice diminue en cas d'impulsion présynaptique seule dans la fenêtre temporelle considérée.

En cas d'impulsion postsynaptique seule dans la fenêtre temporelle considérée, le poids de chaque synapse inhibitrice peut demeurer inchangé. Alternativement, le poids de chaque synapse inhibitrice peut diminuer en cas d'impulsion postsynaptique seule dans la fenêtre temporelle considérée.

Complémentairement au mécanisme d'inhibition latérale de type « le gagnant emporte tout » qui vient d'être décrit, chaque deuxième synapse excitatrice se2 respecte avantageusement une règle STDP qui modifie son poids en fonction des instants auxquels se produisent respectivement les impulsions présynaptiques et les inhibitions latérales. Alternativement, ladite règle STDP peut être implémentée à la fois pour les premières synapses excitatrices se1 et pour les deuxièmes synapses excitatrices se2, ou seulement pour les premières synapses excitatrices se1. On définit typiquement, pour chaque synapse excitatrice, une fenêtre temporelle de coïncidence entre excitation et inhibition, ainsi que précédemment décrit.

Selon un mode de réalisation, la règle STDP implémentée pour chaque synapse excitatrice est la suivante :

- le poids d'une synapse excitatrice demeure inchangé lorsque sa fenêtre temporelle prédéfinie comporte une impulsion présynaptique seule ;
- le poids d'une synapse excitatrice est diminué, par exemple d'une quantité $a_{post}$, lorsque sa fenêtre temporelle prédéfinie comporte une impulsion postsynaptique seule ;
- le poids d'une synapse excitatrice est augmenté, par exemple d'une quantité ($a_{pair}$ - $a_{post}$), lorsque sa fenêtre temporelle prédéfinie comporte une impulsion présynaptique et une impulsion postsynaptique.

Selon ce mode de réalisation, le poids de chaque synapse excitatrice demeure compris entre 0 et une valeur maximale qui est typiquement 1 pour des poids normalisés.

Alternativement, le poids d'une synapse excitatrice peut n'être augmenté que lorsque sa fenêtre temporelle prédéfinie comporte une impulsion postsynaptique succédant à une impulsion présynaptique. Selon cette alternative, lorsque la fenêtre temporelle prédéfinie comporte une impulsion présynaptique succédant à une impulsion postsynaptique, le poids de la synapse excitatrice considérée demeure inchangé ou est diminué.

Pour chaque neurone artificiel n13 de la couche intermédiaire, la quantité $a_{pair}$ est préférentiellement comprise dans l'intervalle [0,001 ; 0,01] et la quantité $a_{post}$ est préférentiellement comprise dans l'intervalle [0,4*$a_{pair}$ ; 0.8*$a_{pair}$]. Dans l'exemple particulier de réalisation, pour chaque neurone artificiel n13 de la couche intermédiaire, la quantité $a_{pair}$ est de 0,003 et la quantité $a_{post}$ est de 0,65*$a_{pair}$.

Pour chaque neurone artificiel n15 de la couche de sortie, la quantité $a_{pair}$ est préférentiellement comprise dans l'intervalle [0,001 ; 0,02] et la quantité $a_{post}$ est préférentiellement comprise dans l'intervalle [0,4* $a_{pair}$; 0.7*$a_{pair}$]. Dans l'exemple particulier de réalisation, pour chaque neurone artificiel n15 de la couche de sortie, la quantité $a_{pair}$ est de 0,01 et la quantité $a_{post}$ est de 0,5*$a_{pair}$.

Selon un autre mode de réalisation, la règle STDP implémentée pour chaque synapse excitatrice est la suivante :

- lorsqu'un neurone artificiel de la couche intermédiaire n13 ou de la couche de sortie n15 reçoit, dans la fenêtre temporelle prédéfinie de coïncidence entre excitation et inhibition, une impulsion transmise par une synapse excitatrice et une inhibition latérale transmise par un autre neurone artificiel de la même couche, le poids de ladite synapse excitatrice est diminué d'une quantité ($a_{lat}$ - $a_{pre}$) ;

- lorsqu'un neurone artificiel de la couche intermédiaire n13 ou de la couche de sortie n15 reçoit, dans la fenêtre temporelle prédéfinie de coïncidence entre excitation et inhibition, une impulsion transmise par une synapse excitatrice et aucune inhibition latérale transmise par un autre neurone artificiel, le poids de ladite synapse excitatrice est augmenté d'une quantité $a_{pre}$.

Pour chaque neurone artificiel n15 de la couche de sortie, la quantité $a_{lat}$ est préférentiellement comprise dans l'intervalle [0 ; 0,2*$a_{pair}$] et la quantité $a_{pre}$ est préférentiellement comprise dans l'intervalle [0 ; 0.5*$a_{lat}$]. Dans l'exemple particulier de réalisation, pour chaque neurone artificiel n15 de la couche de sortie, la quantité $a_{lat}$ est de 0,1*$a_{pair}$ et la quantité $a_{pre}$ est de 0,01*$a_{pair}$.

Selon un autre mode de réalisation, les deux règles STDP qui viennent d'être décrites peuvent être simultanément implémentées. Dans ce cas, chaque synapse excitatrice a une première fenêtre temporelle prédéfinie pour l'implémentation de la première règle STDP et une deuxième fenêtre temporelle prédéfinie pour l'implémentation de la deuxième règle STDP, et les première et deuxième fenêtres temporelles prédéfinies pour chaque synapse excitatrice peuvent être identiques ou distinctes.

Le nombre de neurones artificiels n13 de la couche intermédiaire est choisi suffisamment grand pour que tous les motifs possibles puissent être appris. Le nombre de neurones artificiels n13 de la couche intermédiaire dépend donc du nombre de types différents de potentiels d'action à détecter et du délai de latence paramétré pour la couche intermédiaire 13. Un nombre trop important de neurones artificiels n13 ne modifie pas le fonctionnement du réseau de neurones artificiels mais présente un inconvénient en termes de temps de calcul. Un nombre trop faible de neurones artificiels n13 limite les possibilités d'apprentissage de motifs. En pratique, le nombre de neurones artificiels n13 de la couche intermédiaire est préférentiellement compris dans l'intervalle [30 ; 200]. Dans l'exemple particulier d'implémentation, la couche intermédiaire comporte 80 neurones artificiels n13.

Le nombre de neurones artificiels n15 de la couche de sortie est choisi supérieur ou égal au nombre de types différents de potentiels d'action à détecter. En pratique, le nombre de potentiels d'action à détecter n'étant généralement pas connu d'avance, le nombre de neurones artificiels n15 de la couche de sortie est préférentiellement compris dans l'intervalle [3 ; 30] et plus préférentiellement compris dans l'intervalle [7 ; 15]. Dans l'exemple particulier d'implémentation, la couche de sortie comporte 10 neurones artificiels n15.

Les neurones artificiels n13 de la couche intermédiaire respectent avantageusement une règle de plasticité à court terme STP (de l'anglais « short term plasticity ») qui est à présent décrite. Le rôle des neurones artificiels n11 de la couche d'entrée est de convertir le signal électrique dln sous une forme exploitable par les neurones artificiels n13 de la couche intermédiaire. Les neurones artificiels n11 de la couche d'entrée convertissent ainsi le signal électrique dln en un train de premières impulsions. Le rôle des neurones artificiels n13 de la couche intermédiaire est ensuite d'apprendre à reconnaître des formes d'onde ou des parties de formes d'onde. Pour cela, on souhaite que les neurones artificiels n13 de la couche intermédiaire parviennent à filtrer le bruit afin qu'ils n'apprennent pas à reconnaître du bruit. La règle de plasticité à court terme STP contribue à cet objectif.

On considère chaque première synapse excitatrice se1, qui connecte un neurone artificiel n11 de la couche d'entrée, dit neurone artificiel présynaptique, à un neurone artificiel n13 de la couche intermédiaire, dit neurone artificiel postsynaptique. Selon la règle STP, le poids de chaque première synapse excitatrice se1 est affecté d'un coefficient synaptique $P_{rel}$ tel que $0 \leq P_{rel} \leq 1$.

Chaque neurone artificiel postsynaptique n13 recevant une impulsion via une première synapse excitatrice se1 est excité proportionnellement au produit du coefficient synaptique $P_{rel}$ et du poids de ladite première synapse excitatrice. Le coefficient synaptique $P_{rel}$ diminue lorsque le neurone artificiel présynaptique n11 émet une impulsion, et le coefficient synaptique $P_{rel}$ augmente vers 1 sinon. Selon un mode de réalisation, le coefficient synaptique respecte l'équation suivante :

$$\frac{dP_{rel}}{dt} = \frac{1}{\tau_{stp}}(1 - P_{rel}) - \sum_s P_{rel} * f_d \delta(t - t_s)$$

où s indexe les impulsions présynaptiques, c'est-à-dire les impulsions émises par les neurones artificiels présynaptiques n11, chaque impulsion présynaptique ayant lieu à un instant $t_s$. A chaque fois qu'un neurone artificiel postsynaptique n13 reçoit une impulsion via une première synapse excitatrice se1, le coefficient synaptique $P_{rel}$ de ladite première synapse excitatrice se1 est diminué de la quantité $P_{rel}*f_d$, où $f_d$ est le degré de dépression. Le degré de dépression $f_d$ est choisi de sorte qu'un neurone artificiel n11 de la couche d'entrée qui décharge à chaque pas de temps ait un faible

coefficient synaptique $P_{rel}$. Lorsque la première synapse excitatrice se1 ne transmet aucune impulsion, son coefficient synaptique $P_{rel}$ tend vers 1 dans un temps caractéristique $T_{stp}$. Le temps caractéristique $T_{stp}$ est préférentiellement compris entre 0,1 ms et 10 ms et plus préférentiellement compris entre 0,5 ms et 3 ms. Le temps caractéristique $T_{stp}$ est typiquement de l'ordre de grandeur de la durée d'un potentiel d'action. Dans l'exemple particulier d'implémentation, le temps caractéristique $T_{stp}$ est égal à 1,5 ms. Dans l'exemple particulier d'implémentation, le degré de dépression $f_d$ est choisi tel que :

$$f_d = 1 - \exp(-6,5 * \frac{dt}{\tau_{stp}})$$

La règle de plasticité à court terme STP utilise le fait que les neurones artificiels n11 de la couche d'entrée associés aux valeurs d'amplitudes de bruit ont tendance à émettre des impulsions en permanence : grâce à la règle de plasticité à court terme STP, plus un neurone artificiel n11 de la couche d'entrée émet d'impulsions, plus on diminue la capacité d'excitation de chaque première synapse excitatrice se1 connectant ce neurone artificiel n11 de la couche d'entrée aux neurones artificiels n13 de la couche intermédiaire. Selon le modèle LIF qui est utilisé pour définir le comportement des neurones artificiels n13 de la couche intermédiaire, le potentiel de chaque neurone artificiel n13 augmente lors de la réception d'impulsions via des synapses excitatrices, et tend vers une valeur de repos sinon. Lorsque le signal électrique dln ne contient que du bruit, qui est converti par la couche d'entrée 11 en un train de premières impulsions, l'excitation des neurones artificiels n13 de la couche intermédiaire est à peu près constante, et relativement faible car $P_{rel}$ est faible, et le potentiel des neurones artificiels n13 de la couche intermédiaire se stabilise autour d'une valeur supérieure à la valeur de repos. Le seuil des neurones artificiels n13 de la couche intermédiaire est choisi supérieur à cette valeur de stabilisation, afin que les neurones artificiels n13 de la couche intermédiaire n'émettent pas d'impulsion lorsqu'ils reçoivent du bruit. Dans le même temps, le seuil des neurones artificiels n13 de la couche intermédiaire est choisi de manière que l'augmentation de potentiel liée à l'arrivée d'un potentiel d'action, et donc à l'émission par la couche d'entrée d'une série d'impulsions avec une plus grande capacité d'excitation car $P_{rel}$ est grand, entraîne l'émission d'au moins une impulsion.

Les différents potentiels d'action à détecter et trier présentent généralement des durées différentes et des amplitudes différentes. En conséquence, deux potentiels d'action distincts entraînent typiquement l'activation de différents groupes de neurones artificiels n11 de la couche d'entrée et la transmission aux neurones artificiels n13 de la couche intermédiaire puis aux neurones artificiels n15 de la couche de sortie de trains d'impulsions de tailles différentes, c'est-à-dire ne comportant pas le même nombre d'impulsions. Le seuil d'émission d'impulsion des neurones artificiels n15 de la couche de sortie doit être paramétré suffisamment bas pour que des formes d'onde de courte durée, pour lesquelles le nombre d'impulsions émises par les neurones artificiels de la couche intermédiaire n13 est faible, puissent être détectées. Cependant, si le seuil d'émission d'impulsion des neurones artificiels n15 de la couche de sortie est défini à une valeur basse permettant de détecter des formes d'onde de courte durée, les neurones artificiels n15 de la couche de sortie qui apprennent à reconnaître des formes d'onde de longue durée correspondant à des trains d'impulsions de grande taille vont émettre des impulsions avant que les trains d'impulsions n'aient fini de leur être transmis par les neurones artificiels n13 de la couche intermédiaire. Dans ce cas, comme chaque long train d'impulsions va terminer d'être transmis après qu'un neurone artificiel n15 de la couche de sortie ait émis une impulsion, un autre neurone artificiel n15 de la couche de sortie va pouvoir apprendre à reconnaître la fin de la forme d'onde correspondant au long train d'impulsions. Afin de prévenir ce comportement indésirable, il est possible de définir une forte inhibition latérale. Toutefois, une inhibition latérale trop forte n'est pas souhaitable car différents potentiels d'action peuvent avoir lieu l'un après l'autre dans un bref intervalle de temps : dans un tel cas, on souhaite que les neurones artificiels n15 de la couche de sortie soient capables d'émettre des impulsions l'un après l'autre dans un intervalle de temps suffisamment court. De plus, même si on définit une inhibition latérale suffisamment forte pour garantir qu'un seul neurone artificiel n15 de la couche de sortie n'émet une impulsion pour une forme d'onde donnée, seul le début des longs trains d'impulsions sera pris en compte ce qui empêchera de différencier deux formes d'ondes avant un début identique et une fin différente.

On souhaite que le procédé de tri selon un aspect de l'invention soit robuste à de telles variations d'amplitude et de durée des potentiels d'action, afin que la couche de sortie 15 n'émette toujours qu'une unique impulsion pour chaque occurrence de chaque type de potentiel d'action détecté tout en étant capable de détecter tous les types de potentiel d'action, indépendamment de leur longueur.

Pour ce faire, on introduit avantageusement une règle de plasticité sur le seuil Th d'émission d'impulsion de chaque neurone artificiel n15 de la couche de sortie, afin que le seuil d'émission d'impulsion de chaque neurone artificiel n15 de la couche de sortie s'adapte à la forme d'onde qu'il est en train d'apprendre, et donc à la taille du train d'impulsions correspondant à la forme d'onde qu'il est en train d'apprendre. Lorsqu'une règle de plasticité est implémentée sur le seuil Th de chaque neurone artificiel n15 de la couche de sortie 15, le seuil Th initial est préférentiellement choisi très bas, c'est-à-dire par exemple égal à 1. Le seuil Th s'ajuste ensuite en fonction de sa règle de plasticité. En l'absence d'implémentation d'une telle règle de plasticité, le seuil Th de chaque neurone artificiel n15 de la couche de sortie 15 est préférentiellement choisi dans l'intervalle [3 ; 10]. Deux implémentations possibles de la règle de plasticité sur le seuil Th de chaque neurone artificiel de la couche de sortie sont à présent décrites.

Selon une première variante, on définit une fenêtre temporelle autour de chaque impulsion postsynaptique émise par un neurone artificiel n15 de la couche de sortie et la règle de plasticité sur le seuil d'émission d'impulsion de chaque neurone artificiel n15 de la couche de sortie est la suivante :

- la valeur de seuil de chaque neurone artificiel n15 de la couche de sortie émettant une impulsion à un instant t est augmentée proportionnellement au nombre d'impulsions Ni reçues par ledit neurone artificiel n15 dans la fenêtre temporelle définie : $Th(t) = Ni * \Delta_{Th+}$ ;
- la valeur de seuil de chaque neurone artificiel n15 de la couche de sortie émettant une impulsion à un instant t1 est diminuée de manière que :

$$Th(t2) = \alpha * Th(t1)$$

avec Th(t1) la valeur de seuil à l'instant t1, Th(t2) la valeur de seuil à un instant t2 postérieur à l'instant t1 et $\alpha$ un nombre réel tel que $0 < \alpha < 1$.

$\alpha$ appartient préférentiellement à l'intervalle [0,95 ; 0,999] ; dans l'exemple particulier d'implémentation, $\alpha$ est égal à 0,99. La fenêtre temporelle autour de l'instant t peut être comprise entre 0,1 ms et 20 ms, préférentiellement entre 1 et 3 ms, et est typiquement de 2 ms autour de l'instant t.

$\Delta_{Th+}$ appartient préférentiellement à l'intervalle [0,5*(1-$\alpha$) ; 0,8*(1-$\alpha$)]. Dans l'exemple particulier d'implémentation, $\Delta_{Th+}$ est égal 0,6*(1-$\alpha$).

En conséquence, lorsqu'un potentiel d'action donné se produit, le neurone artificiel n15 de la couche de sortie apprenant à reconnaître ce potentiel d'action reçoit un train de N impulsions présynaptiques, ce qui conduit à une augmentation de son seuil d'émission d'impulsion proportionnelle à N, et émet une impulsion postsynaptique, ce qui conduit à une diminution de son seuil d'émission d'impulsion proportionnelle à la valeur du seuil d'émission d'impulsion. Le seuil d'émission d'impulsion tend ainsi vers une valeur qui est proportionnelle au nombre moyen d'impulsions présynaptiques. On souhaite qu'une majorité des impulsions présynaptiques soit prise en compte avant d'émettre une impulsion postsynaptique. Il peut toutefois arriver que certaines impulsions présynaptiques, habituellement présentes, soient manquantes. Afin de pouvoir émettre une impulsion postsynaptique même si seulement une partie des impulsions présynaptiques attendues est présente, le seuil d'émission d'impulsion ne doit donc pas être trop haut. Il s'agit donc de trouver un compromis entre prendre en compte la majorité des impulsions présynaptiques et être robuste aux éventuelles erreurs de la couche intermédiaire 13. En pratique, on choisit préférentiellement un seuil d'émission d'impulsion valant entre 0,3 et 0,8 fois le nombre moyen d'impulsions présynaptiques émises lors de l'occurrence d'un potentiel d'action.

Selon une deuxième variante, on définit une première fenêtre temporelle avant chaque impulsion postsynaptique et une deuxième fenêtre temporelle après chaque impulsion postsynaptique, et la règle de plasticité sur le seuil d'émission d'impulsion de chaque neurone artificiel n15 de la couche de sortie est la suivante :

- lorsqu'un neurone artificiel n15 de la couche de sortie reçoit une impulsion présynaptique dans la première fenêtre temporelle prédéfinie, le seuil d'émission d'impulsion dudit neurone artificiel n15 de la couche de sortie est diminué ;
- lorsqu'un neurone artificiel n15 de la couche de sortie reçoit une impulsion présynaptique dans la deuxième fenêtre temporelle prédéfinie, le seuil d'émission d'impulsion dudit neurone artificiel n15 de la couche de sortie est augmenté.

La première fenêtre temporelle avant chaque impulsion postsynaptique peut être comprise entre 0,1 ms et 20 ms, préférentiellement entre 1 et 3 ms, et est typiquement de 2 ms. La deuxième fenêtre temporelle après chaque impulsion postsynaptique peut être comprise entre 0,1 ms et 20 ms, préférentiellement entre 1 et 3 ms, et est typiquement de 2 ms.

En conséquence, le seuil d'émission d'impulsion de chaque neurone artificiel n15 de la couche de sortie s'adapte de manière que chaque neurone artificiel n15 de la couche de sortie émet une impulsion postsynaptique après avoir reçu un nombre d'impulsions présynaptiques proportionnel au nombre total d'impulsions présynaptiques reçues pendant la durée de la forme d'onde correspondante. Comme précédemment, il s'agit de trouver un compromis entre prendre en compte la majorité des impulsions présynaptiques et être robuste aux éventuelles erreurs de la couche intermédiaire 13. En pratique, on choisit préférentiellement un seuil d'émission d'impulsion valant entre 0,3 et 0,8 fois la totalité des impulsions présynaptiques émises lors de l'occurrence d'un potentiel d'action.

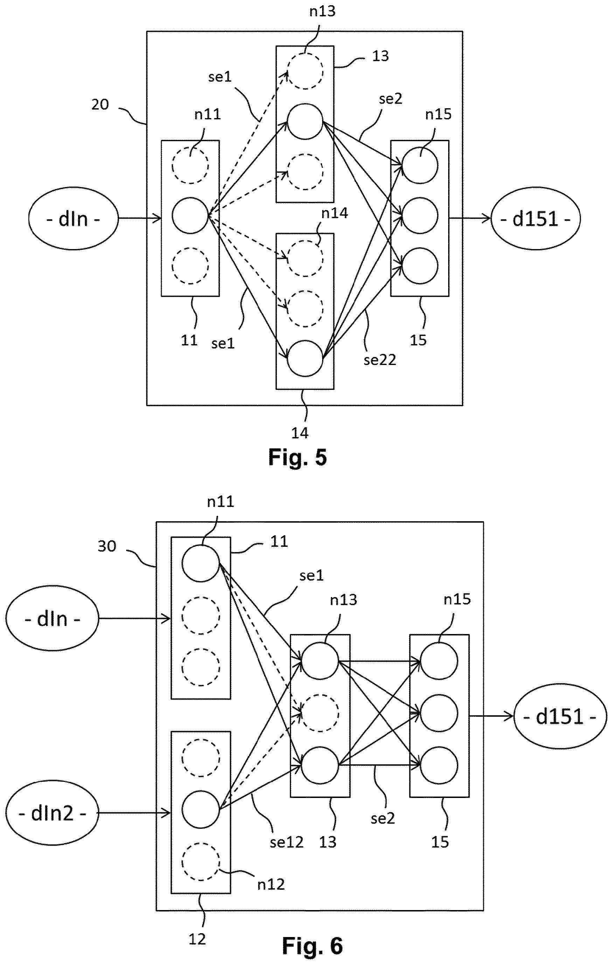

On décrit à présent, en lien avec les figures 5 à 8, plusieurs variantes de réseaux de neurones artificiels pour la mise en oeuvre du procédé 100 de tri non supervisé, en temps réel, de potentiels d'action d'une pluralité de neurones biologiques selon un aspect de l'invention. La figure 5 montre schématiquement un deuxième réseau de neurones artificiels 20 pour la mise en oeuvre du procédé 100 selon un aspect de l'invention, comportant deux couches intermédiaires en parallèle. Le deuxième réseau de neurones artificiels 20 comporte :

- la couche d'entrée 11 comportant la pluralité de neurones artificiels n11,
- la couche intermédiaire 13 comportant la pluralité de neurones artificiels n13,
- une deuxième couche intermédiaire 14 comportant une pluralité de neurones artificiels n14, et
- la couche de sortie 15 comportant la pluralité de neurones artificiels n15.

La pluralité de premières synapses excitatrices se1 connecte chaque neurone artificiel de la couche d'entrée n11 à la pluralité de neurones artificiels de la couche intermédiaire n13 et à la pluralité de neurones artificiels de la deuxième couche intermédiaire n14. La pluralité de deuxièmes synapses excitatrices se2 connecte chaque neurone artificiel de la couche intermédiaire n13 à la pluralité de neurones artificiels de la couche de sortie n15. Une deuxième pluralité de deuxièmes synapses excitatrices se22 connecte chaque neurone artificiel de la deuxième couche intermédiaire n14 à la pluralité de neurones artificiels de la couche de sortie n15.

La couche d'entrée 11 transmet le train de premières impulsions à la couche intermédiaire 13 et à la deuxième couche intermédiaire 14 via la pluralité de premières synapses excitatrices se1. La couche intermédiaire 13 convertit le train de premières impulsions en un train de deuxièmes impulsions et transmet le train de deuxièmes impulsions à la couche de sortie 15 via la pluralité de deuxièmes synapses excitatrices se2. La deuxième couche intermédiaire 14 convertit le train de premières impulsions en un deuxième train de deuxièmes impulsions et transmet le deuxième train de deuxièmes impulsions à la couche de sortie 15 via la deuxième pluralité de deuxièmes synapses excitatrices se22. La couche de sortie 15 utilise alors le train de deuxièmes impulsions et le deuxième train de deuxièmes impulsions pour trier chaque occurrence de chaque type de potentiel d'action présente dans le signal électrique dln.

La figure 6 montre schématiquement un troisième réseau de neurones artificiels 30 pour la mise en oeuvre du procédé 100 selon un aspect de l'invention, comportant deux couches d'entrée en parallèle. Le troisième réseau de neurones artificiels 30 comporte :

- la couche d'entrée 11 comportant la pluralité de neurones artificiels n11,
- une deuxième couche d'entrée 12 comportant une pluralité de neurones artificiels n12,
- la couche intermédiaire 13 comportant la pluralité de neurones artificiels n13 et
- la couche de sortie 15 comportant la pluralité de neurones artificiels n15.

La pluralité de premières synapses excitatrices se1 connecte chaque neurone artificiel de la couche d'entrée n11 à la pluralité de neurones artificiels de la couche intermédiaire n13. Une deuxième pluralité de premières synapses excitatrices se12 connecte chaque neurone artificiel de la deuxième couche d'entrée n12 à la pluralité de neurones artificiels de la couche intermédiaire n13. La pluralité de deuxièmes synapses excitatrices se2 connecte chaque neurone artificiel de la couche intermédiaire n13 à la pluralité de neurones artificiels de la couche de sortie n15.

La couche d'entrée 11 reçoit le signal électrique dln et la deuxième couche d'entrée reçoit un deuxième signal électrique dln2. Le deuxième signal électrique dln2 mesure une activité électrique d'une deuxième pluralité de neurones biologiques et présente une amplitude variable en fonction de potentiels d'action émis par la pluralité de deuxièmes neurones biologiques au cours du temps. Le deuxième signal électrique dln2 peut être différent du signal électrique dln ou identique au signal électrique dln. La deuxième pluralité de neurones biologiques dont l'activité électrique est mesurée par le deuxième signal électrique dln2 peut être partiellement ou totalement différente de la pluralité de neurones biologiques dont l'activité électrique est mesurée par le signal électrique dln. Alternativement, la deuxième pluralité de neurones biologiques dont l'activité électrique est mesurée par le deuxième signal électrique dln2 peut être identique à la pluralité de neurones biologiques dont l'activité électrique est mesurée par le signal électrique dln. La couche d'entrée 11 convertit l'amplitude du premier signal électrique dln en un train de premières impulsions et transmet le train de premières impulsions à la couche intermédiaire 13 via la pluralité de premières synapses excitatrices se1. La deuxième couche d'entrée 12 convertit l'amplitude du deuxième signal électrique dln2 en un deuxième train de premières impulsions et transmet le deuxième train de premières impulsions à la couche intermédiaire 13 via la deuxième pluralité de premières synapses excitatrices se12. La couche intermédiaire 13 convertit le train de premières impulsions et le deuxième train de premières impulsions en un train de deuxièmes impulsions et transmet le train de deuxièmes impulsions à la couche de sortie 15 via la pluralité de deuxièmes synapses excitatrices se2. La couche de sortie 15 utilise alors le train de deuxièmes impulsions pour trier chaque occurrence de chaque type de potentiel d'action présente dans le signal électrique dln et dans le deuxième signal électrique dln2.

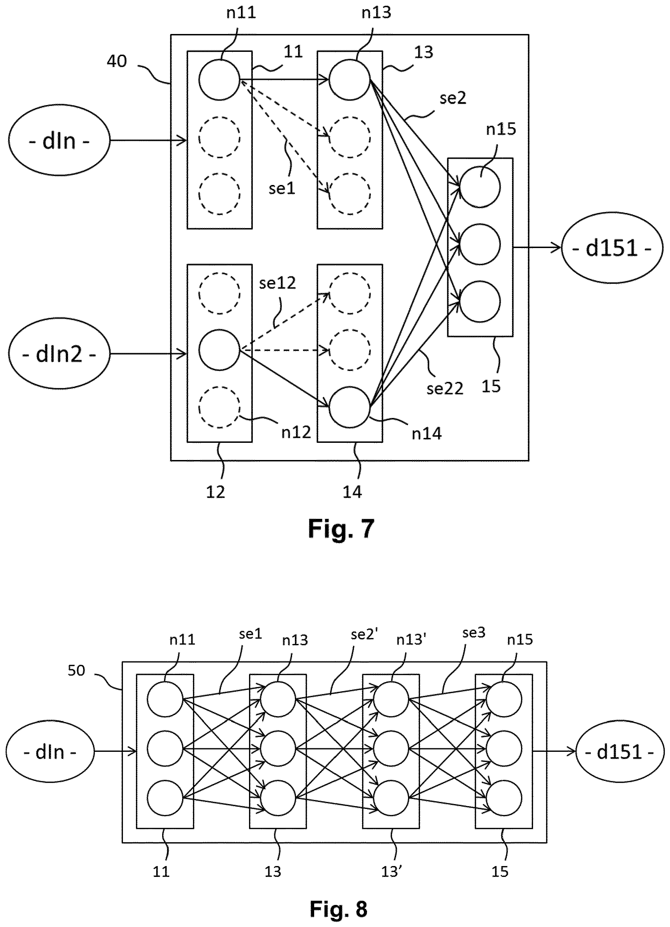

La figure 7 montre schématiquement un quatrième réseau de neurones artificiels 40 pour la mise en oeuvre du procédé 100 selon un aspect de l'invention, comportant deux couches d'entrée en parallèle, chaque couche d'entrée étant associée à sa propre couche intermédiaire. Le quatrième réseau de neurones artificiels 40 comporte :

- la couche d'entrée 11 comportant la pluralité de neurones artificiels n11,
- la deuxième couche d'entrée 12 comportant la pluralité de neurones artificiels n12,
- la couche intermédiaire 13 comportant la pluralité de neurones artificiels n13,
- la deuxième couche intermédiaire 14 comportant la pluralité de neurones artificiels n14, et
- la couche de sortie 15 comportant la pluralité de neurones artificiels n15.

La pluralité de premières synapses excitatrices se1 connecte chaque neurone artificiel de la couche d'entrée n11 à la pluralité de neurones artificiels de la couche intermédiaire n13. Une deuxième pluralité de premières synapses excitatrices se12 connecte chaque neurone artificiel de la deuxième couche d'entrée n12 à la pluralité de neurones artificiels de la deuxième couche intermédiaire n14. La pluralité de deuxièmes synapses excitatrices se2 connecte chaque neurone artificiel de la couche intermédiaire n13 à la pluralité de neurones artificiels de la couche de sortie n15. Une deuxième pluralité de deuxièmes synapses excitatrices se22 connecte chaque neurone artificiel de la deuxième couche intermédiaire n14 à la pluralité de neurones artificiels de la couche de sortie n15.

La couche d'entrée 11 reçoit le signal électrique dln et la deuxième couche d'entrée reçoit le deuxième signal électrique dln2. Le signal électrique dln et le deuxième signal électrique dln2 ont été précédemment décrits. La couche d'entrée 11 convertit l'amplitude du premier signal électrique dln en un train de premières impulsions et transmet le train de premières impulsions à la couche intermédiaire 13 via la pluralité de premières synapses excitatrices se1. La deuxième couche d'entrée 12 convertit l'amplitude du deuxième signal électrique dln2 en un deuxième train de premières impulsions et transmet le deuxième train de premières impulsions à la deuxième couche intermédiaire 14 via la deuxième pluralité de premières synapses excitatrices se12. La couche intermédiaire 13 convertit le train de premières impulsions en un train de deuxièmes impulsions et transmet le train de deuxièmes impulsions à la couche de sortie 15 via la pluralité de deuxièmes synapses excitatrices se2. La deuxième couche intermédiaire 14 convertit le deuxième train de premières impulsions en un deuxième train de deuxièmes impulsions et transmet le deuxième train de deuxièmes impulsions à la couche de sortie 15 via la deuxième pluralité de deuxièmes synapses excitatrices se22. La couche de sortie 15 utilise alors le train de deuxièmes impulsions et le deuxième train de deuxièmes impulsions pour trier chaque occurrence de chaque type de potentiel d'action présente dans le signal électrique dln et dans le deuxième signal électrique dln2.

La figure 8 montre schématiquement un cinquième réseau de neurones artificiels 50 pour la mise en oeuvre du procédé 100 selon un procédé de l'invention, comportant deux couches intermédiaires en série. Le cinquième réseau de neurones artificiels 50 comporte :

- la couche d'entrée 11 comportant la pluralité de neurones artificiels n11,
- la couche intermédiaire 13 comportant la pluralité de neurones artificiels n13,
- une deuxième couche intermédiaire 13' comportant une pluralité de neurones artificiels n13',
- la couche de sortie 15 comportant la pluralité de neurones artificiels n15.

La pluralité de premières synapses excitatrices se1 connecte chaque neurone artificiel de la couche d'entrée n11 à la pluralité de neurones artificiels de la couche intermédiaire n13. Une pluralité de deuxièmes synapses excitatrices se2' connecte chaque neurone artificiel de la couche intermédiaire n13 à la pluralité de neurones artificiels de la deuxième couche intermédiaire n13'. Une pluralité de troisièmes synapses excitatrices se3 connecte chaque neurone artificiel de la deuxième couche intermédiaire n13' à la pluralité de neurones artificiels de la couche de sortie n15.

La couche d'entrée 11 convertit l'amplitude du premier signal électrique dln en un train de premières impulsions et transmet le train de premières impulsions à la couche intermédiaire 13 via la pluralité de premières synapses excitatrices se1. La couche intermédiaire 13 convertit le train de premières impulsions en un train de deuxièmes impulsions et transmet le train de deuxièmes impulsions à la deuxième couche intermédiaire 13' via la pluralité de deuxièmes synapses excitatrices se2'. La deuxième couche intermédiaire 13' convertit le train de deuxièmes impulsions en un train de troisièmes impulsions et transmet le train de troisièmes impulsions à la couche de sortie 15 via la pluralité de troisièmes synapses excitatrices se3. La couche de sortie 15 utilise alors le train de troisièmes impulsions pour trier chaque occurrence de chaque type de potentiel d'action présente dans le signal électrique dln.

**Revendications**

1.   Procédé (100) de tri non supervisé, en temps réel, de potentiels d'action d'une pluralité de neurones biologiques au moyen d'un réseau de neurones artificiels (10, 20, 30, 40, 50) comportant une couche d'entrée (11), une couche intermédiaire (13) et une couche de sortie (15), chaque neurone artificiel de la couche d'entrée (n11) étant connecté à une pluralité de neurones artificiels de la couche intermédiaire (n13) par une pluralité de premières synapses excitatrices (se1) et chaque neurone artificiel de la couche intermédiaire (n13) étant connecté à une pluralité de neurones artificiels de la couche de sortie (n15) par une pluralité de deuxièmes synapses excitatrices (se2), au moins une première ou deuxième synapse excitatrice (se1, se2) connectant un premier neurone artificiel à un deuxième neurone artificiel et ayant un poids qui est modifié en fonction des instants auxquels se produisent des impulsions présynaptiques émises par le premier neurone artificiel et des impulsions postsynaptiques émises par le deuxième neurone artificiel, procédé selon lequel :

- la couche d'entrée (11) reçoit (111) un signal électrique (dln) mesurant une activité électrique d'une pluralité de neurones biologiques, le signal électrique (dln) présentant une amplitude variable en fonction de potentiels d'action émis par la pluralité de neurones biologiques au cours du temps ;
- la couche d'entrée convertit (112) l'amplitude du signal électrique en un train de premières impulsions ;
- la couche d'entrée transmet (113) le train de premières impulsions à la couche intermédiaire ;
- la couche intermédiaire convertit (131) le train de premières impulsions en un train de deuxièmes impulsions ;
- la couche intermédiaire transmet (132) le train de deuxièmes impulsions à la couche de sortie ;
- en fonction du train de deuxièmes impulsions, la couche de sortie trie (151) chaque occurrence de chaque type de potentiel d'action présente dans le signal électrique (dln).

2. Procédé (100) selon la revendication précédente **caractérisé en ce que**, en fonction du train de deuxièmes impulsions, la couche de sortie trie (151) chaque occurrence de chaque type de potentiel d'action présente dans le signal électrique (dln) en activant au plus un unique groupe de neurones artificiels de la couche de sortie pour chaque occurrence d'un potentiel d'action dans le signal électrique (dln), chaque groupe de neurones artificiels de la couche de sortie comportant au moins un neurone artificiel de la couche de sortie et étant associé à un unique type de potentiel d'action et chaque type de potentiel d'action étant associé à un unique groupe de neurones artificiels de la couche de sortie.

3. Procédé (100) selon la revendication précédente dans lequel la couche d'entrée (11) est une matrice de neurones artificiels (n11) ayant :

- Na lignes, le nombre Na de lignes étant choisi en fonction d'une plage de valeurs dans laquelle l'amplitude du signal électrique (dln) est variable, de manière que les neurones artificiels (n11) d'une même ligne s'activent pour une même valeur d'amplitude du signal électrique (dln), et
- Nt colonnes, le nombre Nt de colonnes étant choisi en fonction d'une plage temporelle d'observation du signal électrique (dln), de manière que les neurones artificiels (n11) d'une même colonne reçoivent le signal électrique (dln) à un même instant donné ;

le procédé (100) étant **caractérisé en ce que**, à chaque instant $t_n = n*dt$, avec n un entier naturel et dt un pas temporel :

- une première colonne $C_0$ de neurones artificiels (n11) reçoit le signal électrique (dln), et
- en fonction de la valeur d'amplitude du signal électrique (dln) reçu, au moins un neurone artificiel (n11_1) de la première colonne $C_0$ émet une impulsion qui est transmise à la couche intermédiaire (13).

4. Procédé (100) selon la revendication précédente **caractérisé en ce qu'**une marge (mar) est définie autour de chaque valeur d'amplitude (va) du signal électrique (dln) reçu de manière que, à chaque instant $t_n = n*dt$ :

- la première colonne $C_o$ de neurones artificiels (n11) reçoit le signal électrique (dln), et
- en fonction de la valeur d'amplitude (va) du signal électrique (dln) reçu et de la marge (mar) définie autour de ladite valeur d'amplitude (va), au moins deux neurones artificiels (n11_1) de la première colonne $C_o$ émettent chacun une impulsion, chaque impulsion étant transmise à la couche intermédiaire (13).

5. Procédé (100) selon l'une quelconque des revendications 3 ou 4 **caractérisé en ce que** le nombre de colonne Nt est strictement supérieur à 1 et **en ce que**, à chaque instant $t_k = t_n + k*dt'$, avec dt' un deuxième pas temporel, et pour chaque colonne $C_k$ de neurones artificiels, avec k un entier naturel tel que $1 \leq k \leq Nt-1$, chaque neurone artificiel de même ligne qu'un neurone artificiel de la première colonne $C_o$ ayant émis une impulsion à l'instant $t_n$, émet à son tour une impulsion qui est transmise à la couche intermédiaire (13).

6. Procédé (100) selon l'une quelconque des revendications précédentes **caractérisé en ce que** :

- chaque neurone artificiel de la couche d'entrée (n11) est connecté à une pluralité de neurones artificiels de la couche intermédiaire (n13) par la pluralité de premières synapses excitatrices (se1) de manière qu'une impulsion émise par ledit neurone artificiel de la couche d'entrée (n11) est transmise à la pluralité de neurones artificiels de la couche intermédiaire (n13) par la pluralité de premières synapses excitatrices (se1) ;

- chaque neurone artificiel de la couche intermédiaire (n13) est connecté à une pluralité de neurones artificiels de la couche de sortie (n15) par la pluralité de deuxièmes synapses excitatrices (se2) de manière qu'une impulsion émise par ledit neurone artificiel de la couche intermédiaire (n13) est transmise à la pluralité de neurones artificiels de la couche de sortie (n15) par la pluralité de deuxièmes synapses excitatrices (se2) ;

- chaque neurone artificiel de la couche intermédiaire (n13) et chaque neurone artificiel de la couche de sortie (n15) présentent un potentiel ayant initialement une valeur dite « de repos » ; lorsqu'une synapse excitatrice (se1, se2) transmet une impulsion à un neurone artificiel de la couche intermédiaire (n13) ou de la couche de sortie (n15), le potentiel dudit neurone artificiel (n13, n15) augmente proportionnellement au poids de ladite première synapse excitatrice (se1, se2) ; lorsque le potentiel d'un neurone artificiel de la couche intermédiaire (n13) ou de la couche de sortie (n15) dépasse une valeur de seuil, ledit neurone artificiel (n13, n15) émet une impulsion et son potentiel est rabaissé à une valeur de réinitialisation ;

- en l'absence d'impulsion en entrée d'un neurone artificiel de la couche intermédiaire (n13) ou de la couche de sortie (n15) et lorsque ce neurone (n13, n15) n'émet lui-même pas d'impulsion, le potentiel dudit neurone (n13, n15) revient à sa valeur de repos dans un temps caractéristique $T_m$.

7. Procédé (100) selon la revendication précédente **caractérisé en ce que** le poids de chaque synapse excitatrice (se1, se2) est modifié en fonction d'une fenêtre temporelle prédéfinie :

- pour une synapse excitatrice (se1, se2) donnée, lorsque la fenêtre temporelle prédéfinie comporte une impulsion présynaptique et une impulsion postsynaptique, le poids de cette synapse excitatrice (se1, se2) est augmenté ;

- pour une synapse excitatrice (se1, se2) donnée, lorsque la fenêtre temporelle prédéfinie comporte une impulsion postsynaptique seule, le poids de cette synapse excitatrice (se1, se2) est diminué ;

- pour une synapse excitatrice (se1, se2) donnée, lorsque la fenêtre temporelle prédéfinie comporte une impulsion présynaptique seule, le poids de cette synapse excitatrice (se1, se2) demeure inchangé.

8. Procédé (100) selon l'une quelconque des revendications précédentes **caractérisé en ce que** :

- le réseau de neurones artificiels (10, 20, 30, 40, 50) comporte également au moins un neurone artificiel de détection (nD), chaque neurone artificiel de la couche d'entrée (n11) étant connecté au neurone artificiel de détection (nD) par une première synapse excitatrice de détection (se1D) et le neurone artificiel de détection (nD) étant connecté à une pluralité de neurones artificiels de la couche intermédiaire (n13) par une pluralité de deuxièmes synapses excitatrices de détection (se2D) ;

- chaque première synapse excitatrice de détection (se1 D) présente un poids affecté d'un coefficient synaptique $P_{rel}$ tel que $0 \leq P_{rel} \leq 1$,

  ◦ le coefficient synaptique $P_{rel}$ diminuant lorsque le neurone artificiel présynaptique (n11) émet une impulsion, et
  ◦ le coefficient synaptique $P_{rel}$ augmentant vers 1 sinon avec un temps caractéristique $T_{stp}$ ;

- le neurone artificiel de détection (nD) recevant une impulsion via une première synapse excitatrice de détection (se1D) est excité proportionnellement au produit du coefficient synaptique $P_{rel}$ et du poids de ladite première synapse excitatrice de détection (se1 D).

9. Procédé (100) selon l'une quelconque des revendications précédentes **caractérisé en ce que** :

- chaque neurone artificiel de la couche intermédiaire (n13) est connecté à l'ensemble des autres neurones artificiels de la couche intermédiaire (n13) par une pluralité de premières synapses inhibitrices (si1) de manière qu'une impulsion émise par ledit neurone artificiel de la couche intermédiaire (n13) est transmise à l'ensemble des autres neurones artificiels de la couche intermédiaire (n13) par la pluralité de premières synapses inhibitrices (si1) ;

- chaque neurone artificiel de la couche de sortie (n15) est connecté à l'ensemble des autres neurones artificiels de la couche de sortie (n15) par une pluralité de deuxièmes synapses inhibitrices (si2) de manière qu'une impulsion émise par ledit neurone artificiel de la couche de sortie (n15) est transmise à l'ensemble des autres neurones artificiels de la couche de sortie (n15) par la pluralité de deuxièmes synapses inhibitrices (si2) ;

- lorsqu'une synapse inhibitrice (si1, si2) transmet une impulsion à un neurone artificiel de la couche intermédiaire (n13) ou de la couche de sortie (n15), ledit neurone artificiel (n13, n15) est empêché d'émettre une impulsion pendant une durée prédéfinie dite « période d'inhibition » et/ou le potentiel dudit neurone artificiel (n13, n15) est diminué proportionnellement au poids de ladite synapse inhibitrice (si1, si2).

**10.** Procédé (100) selon la revendication précédente dans lequel une pluralité de deuxièmes synapses excitatrices (se2) connecte chaque neurone artificiel de la couche intermédiaire (n13) à une pluralité de neurones artificiels de la couche de sortie (n15), chaque deuxième synapse excitatrice (se2) ayant un poids, **caractérisé en ce que** :

- pour chaque deuxième synapse excitatrice (se2), lorsqu'un neurone artificiel de la couche de sortie (n15) reçoit, dans une fenêtre temporelle prédéfinie, une impulsion transmise par ladite deuxième synapse excitatrice (se2) et une impulsion transmise par une deuxième synapse inhibitrice (si2), alors le poids de ladite deuxième synapse excitatrice (se2) est diminué ;
- pour chaque deuxième synapse excitatrice (se2), lorsqu'un neurone artificiel de la couche de sortie (n15) reçoit, dans la fenêtre temporelle prédéfinie, une impulsion transmise par ladite deuxième synapse excitatrice (se2) et aucune impulsion transmise par une deuxième synapse inhibitrice (si2), alors le poids de ladite deuxième synapse excitatrice (se2) est augmenté.

**11.** Procédé (100) selon l'une quelconque des revendications précédentes dans lequel :

- chaque neurone artificiel de la couche de sortie (n15) présente un potentiel ;
- chaque neurone artificiel de la couche de sortie (n15) émet une impulsion lorsque son potentiel dépasse une valeur de seuil ;

**caractérisé en ce que** :

- la valeur de seuil de chaque neurone artificiel de la couche de sortie (n15) émettant une impulsion à un instant t est augmentée proportionnellement au nombre d'impulsions reçues par ledit neurone artificiel (n15) dans une fenêtre temporelle autour de l'instant t ;
- la valeur de seuil Th de chaque neurone artificiel de la couche de sortie (n15) émettant une impulsion à un instant t1 est diminuée de manière que :

$$\mathrm{Th}(t2) = \alpha * \mathrm{Th}(t1)$$

avec Th(t1) la valeur de seuil à l'instant t1, Th(t2) la valeur de seuil à un instant t2 postérieur à t1 et $\alpha$ un nombre réel tel que $0 < \alpha < 1$.

**12.** Procédé (100) selon l'une quelconque des revendications précédentes dans lequel le réseau de neurones artificiels (20) comporte la couche d'entrée (11), la couche intermédiaire (13), une deuxième couche intermédiaire (14) et la couche de sortie (15), et selon lequel :

- la couche d'entrée (11) transmet le train de premières impulsions vers la couche intermédiaire (13) et vers la deuxième couche intermédiaire (14) ;
- la deuxième couche intermédiaire (14) convertit le train de premières impulsions en un deuxième train de deuxièmes impulsions ;
- la deuxième couche intermédiaire (14) transmet le deuxième train de deuxièmes impulsions vers la couche de sortie (15) ;
- en fonction du train de deuxièmes impulsions et du deuxième train de deuxièmes impulsions, la couche de sortie (15) trie chaque occurrence de chaque type de potentiel d'action présente dans le signal électrique (dln).

**13.** Procédé (100) selon l'une quelconque des revendications 1 à 11 dans lequel le réseau de neurones artificiels (30) comporte la couche d'entrée (11), une deuxième couche d'entrée (12), la couche intermédiaire (13) et la couche de sortie (15), et selon lequel :

- la deuxième couche d'entrée (12) reçoit un deuxième signal électrique (dln2) mesurant une activité électrique d'une deuxième pluralité de neurones biologiques, le deuxième signal électrique (dln2) présentant une amplitude variable en fonction de potentiels d'action émis par la deuxième pluralité de neurones biologiques au cours du temps ;
- la deuxième couche d'entrée (12) convertit l'amplitude du deuxième signal électrique en un deuxième train de premières impulsions ;
- la deuxième couche d'entrée (12) transmet le deuxième train de premières impulsions à la couche intermédiaire (13) ;
- la couche intermédiaire (13) convertit le train de premières impulsions et le deuxième train de premières impulsions en un train de deuxièmes impulsions.

14. Procédé selon l'une quelconque des revendications 1 à 11 dans lequel le réseau de neurones artificiels (40) comporte la couche d'entrée (11), une deuxième couche d'entrée (12), la couche intermédiaire (13), une deuxième couche intermédiaire (14) et la couche de sortie (15), et selon lequel :

- la deuxième couche d'entrée (12) reçoit un deuxième signal électrique (dln2) mesurant une activité électrique d'une deuxième pluralité de neurones biologiques, le deuxième signal électrique (dln2) présentant une amplitude variable en fonction de potentiels d'action émis par la deuxième pluralité de neurones biologiques au cours du temps ;
- la deuxième couche d'entrée (12) convertit l'amplitude du deuxième signal électrique en un deuxième train de premières impulsions ;
- la deuxième couche d'entrée (12) transmet le deuxième train de premières impulsions à la deuxième couche intermédiaire (14) ;
- la deuxième couche intermédiaire (14) convertit le deuxième train de premières impulsions en un deuxième train de deuxièmes impulsions ;
- la deuxième couche intermédiaire (14) transmet le deuxième train de deuxièmes impulsions à la couche de sortie (15) ;
- en fonction du train de deuxièmes impulsions et du deuxième train de deuxièmes impulsions, la couche de sortie (15) trie chaque occurrence de chaque type de potentiel d'action présente dans le signal électrique (dln).

15. Réseau de neurones artificiels (10, 20, 30, 40, 50) pour la mise en oeuvre d'un procédé (100) de tri non supervisé, en temps réel, de potentiels d'action d'une pluralité de neurones biologiques, le réseau de neurones artificiels (10, 20, 30, 40) comportant :

- une couche d'entrée (11) pour la réception d'un signal électrique (dln) présentant une amplitude variable en fonction de potentiels d'action émis par la pluralité de neurones biologiques au cours du temps, la conversion du signal électrique (dln) en un train de premières impulsions et la transmission du train de premières impulsions à une couche intermédiaire (13) ;
- ladite couche intermédiaire (13) pour la conversion du train de premières impulsions en un train de deuxièmes impulsions et la transmission du train de deuxièmes impulsions à une couche de sortie (15) ;
- ladite couche de sortie (15) pour le tri, en fonction du train de deuxièmes impulsions, de chaque occurrence de chaque type de potentiel d'action présente dans le signal électrique (dln) ;

chaque neurone artificiel de la couche d'entrée (n11) étant connecté à une pluralité de neurones artificiels de la couche intermédiaire (n13) par une pluralité de premières synapses excitatrices (se1) et chaque neurone artificiel de la couche intermédiaire (n13) étant connecté à une pluralité de neurones artificiels de la couche de sortie (n15) par une pluralité de deuxièmes synapses excitatrices (se2), au moins une première ou deuxième synapse excitatrice (se1, se2) connectant un premier neurone artificiel à un deuxième neurone artificiel et ayant un poids qui est modifié en fonction des instants auxquels se produisent des impulsions présynaptiques émises par le premier neurone artificiel et des impulsions postsynaptiques émises par le deuxième neurone artificiel.

**Patentansprüche**

1. Nicht überwachtes Sortierverfahren (100) in Echtzeit von Aktionspotenzialen einer Vielzahl von biologischen Neuronen mittels eines Netzes künstlicher Neuronen (10, 20, 30, 40, 50), umfassend eine Eingangsschicht (11), eine Zwischenschicht (13) und eine Ausgangsschicht (15), wobei jedes künstliche Neuron der Eingangsschicht (n11) an eine Vielzahl von künstlichen Neuronen der Zwischenschicht (n13) durch eine Vielzahl von ersten Erregersynapsen (se1) angeschlossen ist und jedes künstliche Neuron der Zwischenschicht (n13) an eine Vielzahl von künstlichen Neuronen der Ausgangsschicht (n15) durch eine Vielzahl von zweiten Erregersynapsen (se2) angeschlossen ist, wobei wenigstens eine erste oder zweite Erregersynapse (se1, se2) ein erstes künstliches Neuron an ein zweites künstliches Neuron angeschlossen ist und ein Gewicht aufweist, das in Abhängigkeit von den Momenten modifiziert ist, in denen von dem ersten künstlichen Neuron ausgegebene Präsynapsenimpulse und von dem zweiten künstlichen Neuron ausgegebene Postsynapsenimpulse auftreten, wonach gemäß dem Verfahren:

- eine Eingangsschicht (11) ein elektrisches Signal (dln) empfängt (111), das eine elektrische Aktivität einer Vielzahl von biologischen Neuronen misst, wobei das elektrische Signal (dln) eine Amplitude aufweist, die in Abhängigkeit von Aktionspotenzialen variabel ist, die von der Vielzahl von biologischen Neuronen im Verlauf der Zeit ausgegeben werden;
- die Eingangsschicht die Amplitude des elektrischen Signals in einen Zug von ersten Impulsen konvertiert (112);
- die Eingangsschicht den Zug von ersten Impulsen auf die Zwischenschicht überträgt (113);
- die Zwischenschicht den Zug von ersten Impulsen in einen Zug von zweiten Impulsen konvertiert (131);
- die Zwischenschicht den Zug von zweiten Impulsen auf die Ausgangsschicht überträgt (132);
- in Abhängigkeit des Zuges von zweiten Impulsen die Ausgangsschicht jedes Vorkommen jedes Typs von in dem elektrischen Signal (dln) vorhandenem Aktionspotenzial sortiert (151).

2.  Verfahren (100) gemäß dem voranstehenden Anspruch, **dadurch gekennzeichnet, dass** die Ausgangsschicht in Abhängigkeit des Zugs von zweiten Impulsen jedes Vorkommen jedes Typs von dem im elektrischen Signal (dln) vorhandenen Aktionspotenzial durch Aktivieren höchstens einer einzigen Gruppe von künstlichen Neuronen der Ausgangsschicht für jedes Vorkommen eines Aktionspotenzials in dem elektrischen Signal (dln) sortiert (151), wobei jede Gruppe von künstlichen Neuronen der Ausgangsschicht wenigstens ein künstliches Neuron der Ausgangsschicht umfasst und einem einzigen Typ von Aktionspotenzial zugeordnet ist und jeder Typ von Aktionspotenzial einer einzigen Gruppe von künstlichen Neuronen der Ausgangsschicht zugeordnet ist.

3.  Verfahren (100) gemäß dem voranstehenden Anspruch, bei dem die Eingangsschicht (11) eine Matrix aus künstlichen Neuronen (n11) ist, die aufweist:

- Na Zeilen, wobei die Anzahl Na Zeilen in Abhängigkeit von einem Wertebereich ausgewählt ist, in dem die Amplitude des elektrischen Signals (dln) derart variabel ist, dass die künstlichen Neuronen (n11) einer und derselben Linie sich für einen und demselben Amplitudenwert des elektrischen Signals (dln) aktivieren und
- Nt Spalten, wobei die Anzahl Nt Spalten in Abhängigkeit von einem zeitlichen Beobachtungsbereich des elektrischen Signals (dln) derart ausgewählt ist, dass die künstlichen Neuronen (n11) einer und derselben Spalte das elektrische Signal (dln) in einem bestimmten Moment empfangen;

wobei das Verfahren (100) **dadurch gekennzeichnet ist, dass** in jedem Moment $t_n$ = n*dt, wobei n eine ganze natürliche Zahl ist und dt ein zeitlicher Schritt ist:

- eine erste Säule $C_o$ von künstlichen Neuronen (n11) das elektrische Signal (dln) empfängt, und
- in Abhängigkeit von dem Amplitudenwert des empfangenen elektrischen Signals (dln) wenigstens ein künstliches Neuron (n11_1) der ersten Spalte Co einen Impuls ausgibt, der auf die Zwischenschicht (13) übertragen wird.

4.  Verfahren (100) gemäß dem voranstehenden Anspruch, **dadurch gekennzeichnet, dass** eine Marge (mar) um jeden Amplitudenwert (va) des elektrischen Signals (dln) definiert ist, das derart empfangen ist, dass in jedem Moment $t_n$ = n*dt:

- die erste Spalte Co von künstlichen Neuronen (n11) das elektrische Signal (dln) empfängt und
- in Abhängigkeit von dem Amplitudenwert (va) des empfangenen elektrischen Signals (dln) und der um den genannten Amplitudenwert (va) definierten Marge (mar) wenigstens zwei künstliche Neuronen (n1_1) der ersten Spalte Co jeweils einen Impuls ausgeben, wobei jeder Impuls auf die Zwischenschicht (13) übertragen wird.

5.  Verfahren (100) gemäß irgendeinem der Ansprüche 3 oder 4, **dadurch gekennzeichnet, dass** die Spaltenanzahl Nt strikt größer ist als 1 und dass in jedem Moment $t_k$ = tn + k*dt', wobei dt' ein zweiter zeitlicher Schritt ist, und für jede Spalte $C_k$ von künstlichen Neuronen, wobei k eine ganze natürliche Zahl ist, mit $1 \leq k \leq Nt-1$, wobei jedes künstliche Neuron derselben Zeile ein künstliches Neuron der ersten Spalte Co, das einen Impuls im Moment $t_n$ ausgegeben hat, seinerseits einen Impuls ausgibt, der auf die Zwischenschicht (13) übertragen wird.

6.  Verfahren (100) gemäß irgendeinem der voranstehenden Ansprüche, **dadurch gekennzeichnet, dass**:

- jedes künstliche Neuron der Eingangsschicht (n11) an eine Vielzahl von künstlichen Neuronen der Zwischenschicht (n13) durch die Vielzahl von ersten Erregersynapsen (se1) derart angeschlossen ist, dass ein von dem genannten künstlichen Neuron der Eingangsschicht (n11) ausgegebener Impuls auf die Vielzahl von künstlichen Neuronen der Zwischenschicht (n13) durch die Vielzahl von ersten Erregersynapsen (se1) übertragen wird;

- jedes künstliche Neuron der Zwischenschicht (n13) an eine Vielzahl von künstlichen Neuronen der Ausgangsschicht (n15) durch die Vielzahl von zweiten Erregersynapsen (se2) derart angeschlossen ist, dass ein von dem genannten künstlichen Neuron der Zwischenschicht (n13) ausgegebener Impuls auf die Vielzahl von künstlichen Neuronen der Ausgangsschicht (n15) durch die Vielzahl von zweiten Erregersynapsen (se2) übertragen wird;

- jedes künstliche Neuron der Zwischenschicht (n13) und jedes künstliche Neuron der Ausgangsschicht (n15) ein Potenzial aufweisen, das anfänglich einen als "Ruhewert" bezeichneten Wert aufweist; wenn eine Erregersynapse (se1, se2) einen Impuls auf ein künstliches Neuron der Zwischenschicht (n13) oder der Ausgangsschicht (n15) überträgt, erhöht sich das Potenzial des genannten künstlichen Neurons (n13, n15) proportional zu dem Gewicht der genannten Erregersynapse (se1, se2); wenn das Potenzial eines künstlichen Neurons der Zwischenschicht (n13) oder der Ausgangsschicht (n15) einen Schwellenwert übersteigt, gibt das genannte künstliche Neuron (n13, n15) einen Impuls aus und sein Potenzial wird auf einen Reinitialisierungswert abgesenkt;

- bei Fehlen eines Impulses am Eingang eines künstlichen Neurons der Zwischenschicht (n13) oder der Ausgangsschicht (n15) und wenn dieses Neuron (n13, n15) selbst keinen Impuls ausgibt, kehrt das Potenzial des genannten Neurons (n13, n15) innerhalb einer charakteristischen Zeit zu seinem Ruhewert $T_m$ zurück.

**7.** Verfahren (100) gemäß dem voranstehenden Anspruch, **dadurch gekennzeichnet, dass** das Gewicht jeder Erregersynapse (se1, se2) in Abhängigkeit von einem vorbestimmten Zeitfenster modifiziert ist:

- bei einer bestimmten Erregersynapse (se1, se2) ist das Gewicht dieser Erregersynapse (se1, se2) erhöht, wenn das vorbestimmte Zeitfenster einen Präsynapsenimpuls und einen Postsynapsenimpuls umfasst;
- bei einer bestimmten Erregersynapse (se1, se2) ist das Gewicht dieser Erregersynapse (se1, se2) verringert, wenn das vorbestimmte Zeitfenster nur einen Postsynapseimpuls umfasst;
- bei einer bestimmten Erregersynapse (se1, se2) ist das Gewicht dieser Erregersynapse (se1, se2) unverändert geblieben, wenn das vorbestimmte Zeitfenster nur einen Präsynapsenimpuls umfasst.

**8.** Verfahren (100) gemäß irgendeinem der voranstehenden Ansprüche, **dadurch gekennzeichnet, dass**:

- das Netz künstlicher Neuronen (10, 20, 30, 40, 50) ebenfalls wenigstens ein künstliches Detektionsneuron (nD) umfasst, wobei jedes künstliche Neuron der Eingangsschicht (n11) an das künstliche Detektionsneuron (nD) durch eine erste Detektionserregersynapse (se1D) angeschlossen ist und das künstliche Detektionsneuron (nD) an eine Vielzahl von künstlichen Neuronen der Zwischenschicht (n13) durch eine Vielzahl von zweiten Detektionserregersynapsen (se2D) angeschlossen ist;

- jede erste Detektionserregersynapse (se1D) ein Gewicht aufweist, das von einem Synapsenkoeffizienten $P_{rel}$, mit $0 \leq P_{rel} \leq 1$, beeinflusst ist,

  ◦ wobei der Synapsenkoeffizient $P_{rel}$ abnimmt, wenn das künstliche Präsynapseneuron (n11) einen Impuls ausgibt, und
  ◦ wobei der Synapsenkoeffizient $P_{rel}$ auf 1 ansteigt ansonsten mit einer charakteristischen Zeit $T_{stp}$;

- das künstliche Detektionsneuron (nD), das einen Impuls über eine erste Detektionserregersynapse (se1D) empfängt, proportional zu dem Produkt des Synapsenkoeffizienten $P_{rel}$ und dem Gewicht der genannten ersten Detektionserregersynapse (se1 D) erregt ist.

**9.** Verfahren (100) gemäß irgendeinem der voranstehenden Ansprüche, **dadurch gekennzeichnet, dass**:

- jedes künstliche Neuron der Zwischenschicht (n13) an die Gruppe der anderen künstlichen Neuronen der Zwischenschicht (n13) durch eine Vielzahl von ersten Inhibitorsynapsen (si1) derart angeschlossen ist, dass ein von dem genannten künstlichen Neuron der Zwischenschicht (n13) ausgegebener Impuls auf die Gruppe der anderen künstlichen Neuronen der Zwischenschicht (n13) durch die Vielzahl von ersten Inhibitorsynapsen (si1) übertragen wird;

- jedes künstliche Neuron der Ausgangsschicht (n15) an die Gruppe der anderen künstlichen Neuronen der Ausgangsschicht (n15) durch eine Vielzahl von zweiten Inhibitorsynapsen (si2) derart angeschlossen ist, dass ein von dem genannten künstlichen Neuron der Ausgangsschicht (n15) ausgegebener Impuls auf die Gruppe der anderen künstlichen Neuronen der Ausgangsschicht (n15) durch die Vielzahl von zweiten Inhibitorsynapsen (si2) übertragen wird;
- wenn eine Inhibitorsynapse (si1, si2) einen Impuls auf ein künstliches Neuron der Zwischenschicht (n13) oder der Ausgangsschicht (n15) überträgt, wird das genannte künstliche Neuron (n13, n15) gehindert, einen Impuls während einer vorbestimmten, als "Inhibitionsperiode" bezeichneten Dauer auszugeben, und / oder das Potenzial des genannten künstlichen Neurons (n13, n15) wird proportional zu dem Gewicht der genannten Inhibitorsynapse (si1, si2) gemindert.

**10.** Verfahren (100) gemäß dem voranstehenden Anspruch, bei dem eine Vielzahl von zweiten Erregersynapsen (se2) jedes künstliche Neuron der Zwischenschicht (n13) an eine Vielzahl von künstlichen Neuronen der Ausgangsschicht (n15) anschließt, wobei jede zweite Erregersynapse (se2) ein Gewicht hat, **dadurch gekennzeichnet, dass**:

- wenn ein künstliches Neuron der Ausgangsschicht (n15) in einem vorbestimmten Zeitfenster einen von der genannten zweiten Erregersynapse (se2) und einen von einer zweiten Inhibitorsynapse (si2) übertragenen Impuls empfängt, das Gewicht der genannten zweiten Erregersynapse (se2) bei jeder zweiten Erregersynapse (se2) gemindert ist;
- wenn ein künstliches Neuron der Ausgangsschicht (n15) in dem vorbestimmten Zeitfenster einen von der genannten zweiten Erregersynapse (se2) übertragenen Impuls empfängt und keinen von einer zweiten Inhibitorsynapse (si2) übertragenen Impuls empfängt, das Gewicht der genannten zweiten Erregersynapse (se2) erhöht ist.

**11.** Verfahren (100) gemäß irgendeinem der voranstehenden Ansprüche, bei dem:

- jedes künstliche Neuron der Ausgangsschicht (n15) ein Potenzial aufweist;
- jedes künstliche Neuron der Ausgangsschicht (n15) einen Impuls ausgibt, wenn sein Potenzial einen Schwellenwert übersteigt;

**dadurch gekennzeichnet, dass**:

- der Schwellenwert jedes künstlichen Neurons der Ausgangsschicht (n15), das einen Impuls zu einem Moment t ausgibt, proportional zur Anzahl von Impulsen erhöht ist, die von dem genannten künstlichen Neuron (n15) in einem Zeitfenster um den Moment t empfangen sind;
- der Schwellenwert Th jedes künstlichen Neurons der Ausgangsschicht (n15), das einen Impuls in einem Moment t1 ausgibt, derart gemindert ist, dass

$$Th\,(t2) = \alpha * Th\,(t1),$$

wobei Th (t1) der Schwellenwert im Moment t1 ist, Th (t2) der Schwellenwert in einem Moment t2 nach t1 ist und $\alpha$ eine reelle Zahl, mit $0 < \alpha < 1$ ist.

**12.** Verfahren (100) gemäß irgendeinem der voranstehenden Ansprüche, bei dem das Netz künstlicher Neuronen (20) die Eingangsschicht (11), die Zwischenschicht (13), eine zweite Zwischenschicht (14) und die Ausgangsschicht (15) umfasst und gemäß dem:

- die Eingangsschicht (11) den Zug von ersten Impulsen auf die Zwischenschicht (13) und auf die zweite Zwischenschicht (14) überträgt;
- die zweite Zwischenschicht (14) den Zug von ersten Impulsen in einen zweiten Zug von zweiten Impulsen konvertiert;
- die zweite Zwischenschicht (14) den zweiten Zug von zweiten Impulsen auf die Ausgangsschicht (15) überträgt;
- die Ausgangsschicht (15) in Abhängigkeit des Zugs von zweiten Impulsen und des zweiten Zugs von zweiten Impulsen jedes Vorkommen von jedem Typ von im elektrischen Signal vorhandenen Aktionspotenzial sortiert.

**13.** Verfahren (100) gemäß irgendeinem der Ansprüche 1 bis 11, bei dem das Netz künstlicher Neuronen (30) die Eingangsschicht (11), eine zweite Eingangsschicht (12), die Zwischenschicht (13) und die Ausgangsschicht (15) umfasst, und gemäß dem:

- die zweite Eingangsschicht (12) ein zweites elektrisches Signal (dln2) empfängt, das eine elektrische Aktivität einer zweiten Vielzahl von biologischen Neuronen misst, wobei das zweite elektrische Signal (dln2) eine Amplitude aufweist, die in Abhängigkeit von Aktionspotenzialen variabel ist, die von der zweiten Vielzahl von biologischen Neuronen im Verlauf der Zeit ausgegeben ist;

- die zweite Eingangsschicht (12) die Amplitude des zweiten elektrischen Signals in einen zweiten Zug von ersten Impulsen konvertiert;

- die zweite Eingangsschicht (12) den zweiten Zug von ersten Impulsen auf die Zwischenschicht (13) überträgt;

- die Zwischenschicht (13) den Zug von ersten Impulsen und den zweiten Zug von ersten Impulsen in einen Zug von zweiten Impulsen konvertiert.

14. Verfahren gemäß irgendeinem der Ansprüche 1 bis 11, bei dem das Netz künstlicher Neuronen (40) die Eingangsschicht (11), eine zweite Eingangsschicht (12), die Zwischenschicht (13), eine zweite Zwischenschicht (14) und die Ausgangsschicht (15) umfasst und gemäß der:

- die zweite Eingangsschicht (12) ein zweites elektrisches Signal (dln2) empfängt, das eine elektrische Aktivität einer zweiten Vielzahl von biologischen Neuronen misst, wobei das zweite elektrische Signals (dln2) eine Amplitude aufweist, die in Abhängigkeit von Aktionspotenzialen variabel ist, die von der zweiten Vielzahl von biologischen Neuronen im Verlauf der Zeit ausgegeben sind;

- die zweie Eingangsschicht (12) die Amplitude des zweiten elektrischen Signals in einen zweiten Zug von ersten Impulsen konvertiert;

- die zweite Eingangsschicht (12) den zweiten Zug von ersten Impulsen auf die zweite Zwischenschicht (14) überträgt;

- die zweite Zwischenschicht (14) den zweiten Zug von ersten Impulsen in einen zweiten Zug von zweiten Impulsen konvertiert;

- die zweite Zwischenschicht (14) den zweiten Zug von zweiten Impulsen auf die Ausgangsschicht (15) überträgt;

- die Ausgangsschicht (15) in Abhängigkeit des Zugs von zweiten Impulsen und des zweiten Zugs von zweiten Impulsen jedes Vorkommen jedes Typs von in dem elektrischen Signal (dln) vorhandenen Aktionspotenzials sortiert.

15. Netz künstlicher Neuronen (10, 20, 30, 40, 50) für die Umsetzung eines nicht überwachten Sortierverfahrens (100) in Echtzeit von Aktionspotenzialen einer Vielzahl von biologischen Neuronen, wobei das Netz künstlicher Neuronen (10, 20, 30, 40, 50) umfasst

- eine Eingangsschicht (11) für den Empfang eines elektrischen Signals (dln), das eine Amplitude aufweist, die in Abhängigkeit von Aktionspotenzialen variabel sind, die von der Vielzahl von biologischen Neuronen im Verlauf der Zeit ausgegeben werden, die Konvertierung des elektrischen Signals (dln) in einen Zug von ersten Impulsen und die Übertragung des Zugs von ersten Impulsen auf eine Zwischenschicht (13);

- die genannte Zwischenschicht (13) für die Konvertierung des Zugs von ersten Impulsen in einen Zug von zweiten Impulsen und die Übertragung des Zugs von zweiten Impulsen auf eine Ausgangsschicht (15);

- die genannte Ausgangsschicht (15) zum Sortieren jedes Vorkommens jedes Typs von in dem elektrischen Signal (dln) vorhandenen Aktionspotenzials in Abhängigkeit des Zugs von zweiten Impulsen;

wobei jedes künstliche Neuron der Eingangsschicht (n11) an eine Vielzahl von künstlichen Neuronen der Zwischenschicht (n13) durch eine Vielzahl von ersten Erregersynapsen (set1) angeschlossen ist und jedes künstliche Neuron der Zwischenschicht (n13) an eine Vielzahl von künstlichen Neuronen der Ausgangsschicht (n15) durch eine Vielzahl von zweiten Erregersynapsen (se2) angeschlossen ist, wobei wenigstens eine oder zweite Erregersynapse (se1, se2) ein erstes künstliches Neuron an ein zweites künstliches Neuron anschließt und ein Gewicht hat, das in Abhängigkeit von den Momenten modifiziert ist, in denen Präsynapsenimpulse, die von dem ersten künstlichen Neuron ausgegeben sind, und Postsynapsenimpulse, die von dem zweiten künstlichen Neuron ausgegeben sind, auftreten.

**Claims**

1. Method (100) for unsupervised sorting, in real time, of action potentials of a plurality of biological neurons by means of a network of artificial neurons (10, 20, 30, 40, 50) comprising an input layer (11), an intermediate layer (13) and an output layer (15), each artificial neuron of the input layer (n11) being connected to a plurality of artificial neurons of the intermediate layer (n13) by a plurality of first excitatory synapses (se1) and each artificial neuron of the intermediate layer (n13) being connected to a plurality of artificial neurons of the output layer (n15) by a plurality of second excitatory synapses (se2), at least one first or second excitatory synapse (se1, se2) connecting a first artificial neuron to a second artificial neuron and having a weight that is modified as a function of the instants at which take place the presynaptic spikes emitted by the first artificial neuron and the postsynaptic spikes emitted by the second artificial neuron, method according to which:

- the input layer (11) receives (111) an electrical signal (din) measuring an electrical activity of a plurality of biological neurons, the electrical signal (din) having a variable amplitude as a function of action potentials emitted by the plurality of biological neurons over time;
- the input layer converts (112) the amplitude of the electrical signal into a train of first spikes;
- the input layer transmits (113) the train of first spikes to the intermediate layer;
- the intermediate layer converts (131) the train of first spikes into a train of second spikes;
- the intermediate layer transmits (132) the train of second spikes to the output layer;
- as a function of the train of second spikes, the output layer sorts (151) each occurrence of each type of action potential present in the electrical signal (din).

2. Method (100) according to the preceding claim **characterized in that**, as a function of the train of second spikes, the output layer sorts (151) each occurrence of each type of action potential present in the electrical signal (din) by activating at the most a single group of artificial neurons of the output layer for each occurrence of an action potential in the electrical signal (din), each group of artificial neurons of the output layer comprising at least one artificial neuron of the output layer and being associated with a single type of action potential and each type of action potential being associated with a single group of artificial neurons of the output layer.

3. Method (100) according to the preceding claim wherein the input layer (11) is a matrix of artificial neurons (n11) having:

- Na lines, the number Na of lines being chosen as a function of a range of values in which the amplitude of the electrical signal (din) is variable, in such a way that the artificial neurons (n11) of a same line are activated for a same amplitude value of the electrical signal (din), and
- Nt columns, the number Nt of columns being chosen as a function of a time frame for observing the electrical signal (din), in such a way that the artificial neurons (n11) of a same column receive the electrical signal (din) at a same given instant;

the method (100) being **characterized in that**, at each instant $t_n = n*dt$, with n a natural integer and dt a time step:

- a first column Co of artificial neurons (n11) receives the electrical signal (din), and
- as a function of the amplitude value of the electrical signal (din) received, at least one artificial neuron (n11_1) of the first column Co emits a spike that is transmitted to the intermediate layer (13).

4. Method (100) according to the preceding claim **characterized in that** a margin (mar) is defined around each amplitude value (va) of the electrical signal (din) received in such a way that, at each instant $t_n = n*dt$:

- the first column Co of artificial neurons (n11) receives the electrical signal (dln), and
- as a function of the amplitude value (va) of the electrical signal (dln) received and of the margin (mar) defined around said amplitude value (va), at least two artificial neurons (n11_1) of the first column Co each emit a spike, each spike being transmitted to the intermediate layer (13).

5. Method (100) according to any of claims 3 or 4 **characterized in that** the number of columns Nt is strictly greater than 1 and **in that**, at each instant $t_k = t_n + k*dt'$, with dt' a second time step, and for each column $C_k$ of artificial neurons, with k a natural integer such that $1 \leq k \leq Nt-1$, each artificial neuron of same line as an artificial neuron of the first column Co having emitted a spike at the instant $t_n$, emits in its turn a spike that is transmitted to the intermediate layer (13).

6. Method (100) according to any of the preceding claims **characterized in that**:

- each artificial neuron of the input layer (n11) is connected to a plurality of artificial neurons of the intermediate layer (n13) by the plurality of first excitatory synapses (se1) in such a way that a spike emitted by said artificial neuron of the input layer (n11) is transmitted to the plurality of artificial neurons of the intermediate layer (n13) by the plurality of first excitatory synapses (se1);
- each artificial neuron of the intermediate layer (n13) is connected to a plurality of artificial neurons of the output layer (n15) by the plurality of second excitatory synapses (se2) in such a way that a spike emitted by said artificial neuron of the intermediate layer (n13) is transmitted to the plurality of artificial neurons of the output layer (n15) by the plurality of second excitatory synapses (se2);

- each artificial neuron of the intermediate layer (n13) and each artificial neuron of the output layer (n15) have a potential having initially a value designated "rest value"; when an excitatory synapse (se1, se2) transmits a spike to an artificial neuron of the intermediate layer (n13) or of the output layer (n15), the potential of said artificial neuron (n13, n15) increases proportionally to the weight of said first excitatory synapse (se1, se2); when the potential of an artificial neuron of the intermediate layer (n13) or of the output layer (n15) exceeds a threshold value, said artificial neuron (n13, n15) emits a spike and its potential is reduced to a reset value;

- in the absence of spike at the input of an artificial neuron of the intermediate layer (n13) or of the output layer (n15) and when this neuron (n13, n15) does not emit a spike itself, the potential of said neuron (n13, n15) returns to its rest value in a characteristic time $T_m$.

7. Method (100) according to the preceding claim **characterized in that** the weight of each excitatory synapse (se1, se2) is modified as a function of a predefined time window:

- for a given excitatory synapse (se1, se2), when the predefined time window comprises a presynaptic spike and a postsynaptic spike, the weight of this excitatory synapse (se1, se2) is increased;
- for a given excitatory synapse (se1, se2), when the predefined time window comprises a postsynaptic spike only, the weight of this excitatory synapse (se1, se2) is decreased;
- for a given excitatory synapse (se1, se2), when the predefined time window comprises a presynaptic spike only, the weight of this excitatory synapse (se1, se2) remains unchanged.

8. Method (100) according to any of the preceding claims **characterized in that**:

- the network of artificial neurons (10, 20, 30, 40, 50) also comprises at least one artificial detection neuron (nD), each artificial neuron of the input layer (n11) being connected to the artificial detection neuron (nD) by a first excitatory detection synapse (se1D) and the artificial detection neuron (nD) being connected to a plurality of artificial neurons of the intermediate layer (n13) by a plurality of second excitatory detection synapses (se2D);
- each first excitatory detection synapse (se1D) has a weight allotted a synaptic coefficient $P_{rel}$ such that $0 \leq P_{rel} \leq 1$,

    • the synaptic coefficient $P_{rel}$ decreasing when the presynaptic artificial neuron (n11) emits a spike, and
    • the synaptic coefficient $P_{rel}$ increasing towards 1 otherwise with a characteristic time $T_{stp}$;

- the artificial detection neuron (nD) receiving a spike via a first excitatory detection synapse (se1D) is excited proportionally to the product of the synaptic coefficient $P_{rel}$ and the weight of said first excitatory detection synapse (se1D).

9. Method (100) according to any of the preceding claims **characterized in that**:

- each artificial neuron of the intermediate layer (n13) is connected to all of the other artificial neurons of the intermediate layer (n13) by a plurality of first inhibitory synapses (si1) in such a way that a spike emitted by said artificial neuron of the intermediate layer (n13) is transmitted to all of the other artificial neurons of the intermediate layer (n13) by the plurality of first inhibitory synapses (si1);
- each artificial neuron of the output layer (n15) is connected to all of the other artificial neurons of the output layer (n15) by a plurality of second inhibitory synapses (si2) in such a way that a spike emitted by said artificial neuron of the output layer (n15) is transmitted to all of the other artificial neurons of the output layer (n15) by the plurality of second inhibitory synapses (si2);
- when an inhibitory synapse (si1, si2) transmits a spike to an artificial neuron of the intermediate layer (n13) or of the output layer (n15), said artificial neuron (n13, n15) is prevented from emitting a spike for a predefined duration designated "inhibition period" and/or the potential of said artificial neuron (n13, n15) is decreased proportionally to the weight of said inhibitory synapse (si1, si2).

10. Method (100) according to the preceding claim wherein a plurality of second excitatory synapses (se2) connects each artificial neuron of the intermediate layer (n13) to a plurality of artificial neurons of the output layer (n15), each second excitatory synapse (se2) having a weight, **characterized in that**:

- for each second excitatory synapse (se2), when an artificial neuron of the output layer (n15) receives, in a predefined time window, a spike transmitted by said second excitatory synapse (se2) and a spike transmitted by a second inhibitory synapse (si2), then the weight of said second excitatory synapse (se2) is decreased;
- for each second excitatory synapse (se2), when an artificial neuron of the output layer (n15) receives, in the predefined time window, a spike transmitted by said second excitatory synapse (se2) and no spike transmitted by a second inhibitory synapse (si2), then the weight of said second excitatory synapse (se2) is increased.

11. Method (100) according to any of the preceding claims wherein:

- each artificial neuron of the output layer (n15) has a potential;
- each artificial neuron of the output layer (n15) emits a spike when its potential exceeds a threshold value;

**characterized in that**:

- the threshold value of each artificial neuron of the output layer (n15) emitting a spike at an instant t is increased proportionally to the number of spikes received by said artificial neuron (n15) in a time window around the instant t;
- the threshold value Th of each artificial neuron of the output layer (n15) emitting a spike at an instant t1 is decreased in such a way that:

$$Th(t2) = \alpha * Th(t1)$$

with Th(t1) the threshold value at the instant t1, Th(t2) the threshold value at an instant t2 later than t1 and $\alpha$ a real number such that $0 < \alpha < 1$.

12. Method (100) according to any of the preceding claims wherein the network of artificial neurons (20) comprises the input layer (11), the intermediate layer (13), a second intermediate layer (14) and the output layer (15), and according to which:

- the input layer (11) transmits the train of first spikes to the intermediate layer (13) and to the second intermediate layer (14);
- the second intermediate layer (14) converts the train of first spikes into a second train of second spikes;
- the second intermediate layer (14) transmits the second train of second spikes to the output layer (15);
- as a function of the train of second spikes and the second train of second spikes, the output layer (15) sorts each occurrence of each type of action potential present in the electrical signal (dln).

13. Method (100) according to any of claims 1 to 11 wherein the network of artificial neurons (30) comprises the input layer (11), a second input layer (12), the intermediate layer (13) and the output layer (15), and according to which:

- the second input layer (12) receives a second electrical signal (dln2) measuring an electrical activity of a second plurality of biological neurons, the second electrical signal (dln2) having a variable amplitude as a function of action potentials emitted by the second plurality of biological neurons over time;
- the second input layer (12) converts the amplitude of the second electrical signal into a second train of first spikes;
- the second input layer (12) transmits the second train of first spikes to the intermediate layer (13);
- the intermediate layer (13) converts the train of first spikes and the second train of first spikes into a train of second spikes.

14. Method according to any of claims 1 to 11 wherein the network of artificial neurons (40) comprises the input layer (11), a second input layer (12), the intermediate layer (13), a second intermediate layer (14) and the output layer (15), and according to which:

- the second input layer (12) receives a second electrical signal (dln2) measuring an electrical activity of a second plurality of biological neurons, the second electrical signal (dln2) having a variable amplitude as a function of action potentials emitted by the second plurality of biological neurons over time;
- the second input layer (12) converts the amplitude of the second electrical signal into a second train of first spikes;
- the second input layer (12) transmits the second train of first spikes to the second intermediate layer (14);
- the second intermediate layer (14) converts the second train of first spikes into a second train of second spikes;

- the second intermediate layer (14) transmits the second train of second spikes to the output layer (15);
- as a function of the train of second spikes and of the second train of second spikes, the output layer (15) sorts each occurrence of each type of action potential present in the electrical signal (din).

15. Network of artificial neurons (10, 20, 30, 40, 50) for the implementation of a method (100) for unsupervised sorting, in real time, of action potentials of a plurality of biological neurons, the network of artificial neurons (10, 20, 30, 40, 50) comprising:

- an input layer (11) for the reception of an electrical signal (dln)having a variable amplitude as a function of action potentials emitted by the plurality of biological neurons over time, the conversion of the electrical signal (dln) into a train of first spikes and the transmission of the train of first spikes to an intermediate layer (13);
- said intermediate layer (13) for the conversion of the train of first spikes into a train of second spikes and the transmission of the train of second spikes to an output layer (15);
- said output layer (15) for the sorting, as a function of the train of second spikes, of each occurrence of each type of action potential present in the electrical signal (dln);

each artificial neuron of the input layer (n11) being connected to a plurality of artificial neurons of the intermediate layer (n13) by a plurality of first excitatory synapses (se1) and each artificial neuron of the intermediate layer (n13) being connected to a plurality of artificial neurons of the output layer (n15) by a plurality of second excitatory synapses (se2), at least one first or second excitatory synapse (se1, se2) connecting a first artificial neuron to a second artificial neuron and having a weight that is modified as a function of the instants at which take place presynaptic spikes emitted by the first artificial neuron and postsynaptic spikes emitted by the second artificial neuron.

**Fig. 1a**

**Fig. 1b**

Fig. 2a

**Fig. 2b**

**Fig. 2c**

**Fig. 3a**

**Fig. 3b**

**Fig. 3c**

Fig. 4a

Fig. 4b

Fig. 5

Fig. 6

**Fig. 7**

**Fig. 8**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2014025765 A2
- WO 20151834328 A1
- US 20090216091 A

**Littérature non-brevet citée dans la description**

- **ZHANG et al.** A neuromorphic neural spike clustering processor for deep-brain sensing and stimulation systems. *ISLPED,* 2015
- **F. CORRADI ; G. INDIVERI.** A Neuromorphic Event-Based Neural Recording System for Smart Brain-Machine-Interfaces. *IEEE Transactions on Biomedical Circuits and Systems,* Octobre 2015, vol. 9 (5), 699-709